# EUROPEAN PATENT SPECIFICATION

(11) **EP 0 175 266 B1**
(45) Date of publication and mention of the grant of the patent: **20.03.1996**
(21) Application number: 85111454.6
(22) Date of filing: 10.09.1985
(51) Int. Cl.: C07K 5/06, A61K 38/05

(54) **Antihypertensive derivatives**
Antihypertensive Derivate
Dérivés antihypertensifs

(30) Priority: 12.09.1984 US 649797; 08.07.1985 US 752695
(43) Date of publication of application: 26.03.1986
(73) Proprietor: RHONE-POULENC RORER PHARMACEUTICALS INC., Collegeville, PA 19426-0107 (US)
(72) Inventor: Neiss, Edward S., New Canaan Connecticut (US); Suh, John T., Greenwich Connecticut (US); Regan, John R., Larchmont New York 10538 (US); Skiles, Jerry W., Tuckahoe New York (US); Barton, Jeffrey N., New York New York (US); Menard, Paul, Tuckahoe New York (US); Mencel, James J., Norwalk Connecticut (US)
(74) Representative: Grünecker, Kinkeldey, Stockmair & Schwanhäusser Anwaltssozietät

(56) References cited:
- EP-A- 0 031 104
- EP-A- 0 048 159
- EP-A- 0 052 991
- EP-A- 0 059 666
- EP-A- 0 080 822
- EP-A- 0 088 341
- EP-A- 0 088 350
- EP-A- 0 095 584
- EP-A- 0 121 830
- EP-A- 0 127 124
- GB-A- 2 123 834
- GB-A- 2 130 221

## Description

This application relates to compounds, their pharmaceutically acceptable salts, and pharmaceutical preparations made therefrom, having utility in the treatment of hypertension in subjects suffering therefrom.

EP-A-88350 discloses carboxyalkyl dipeptides substituted with groups containing one sulfamoyl group which are useful as antihypertensive agents in the treatment of congestive heart failure and glaucoma.

EP-A-80822 discloses armethyleneoxy substituted peptides havinq antihypertensive activity.

The present invention comprises a compound of the formula (I) 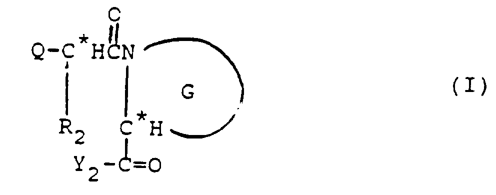 and pharmaceutically acceptable acid addition, alkali and alkaline earth metal salts thereof, wherein
Q is Y₁-CO-C*H(R₁)NH-, R₁-CO-S(C*H(R₁))₀₋₁, or HS-(C*HR₁)₀₋₁; wherein * indicates an asymmetric carbon; Y₁ and Y₂ are independently -OH, -OR, or -NR₁R₂;
G is 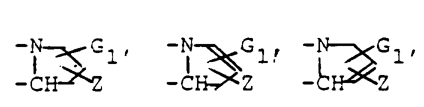 or 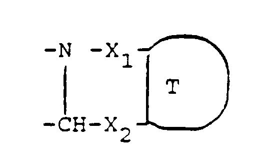
G₁ is H, -OH, C₁₋₆ alkyl or C₁₋₆ alkoxy;
X₁ and X₂ are independently a chemical bond or an alkylene bridge 1, 2 or 3 carbon atoms in length, provided that the ring which contains X₁ and X₂ contains 4 to 6 carbon atoms; one or both of X₁ and X₂ is optionally substituted with -OH, C₁₋₆ alkyl, or C₁₋₆ alkoxy; one of X₁ and X₂ is substituted with Z; X₁ and X₂ are otherwise substituted with hydrogen; and T is a saturated, unsaturated or aromatic hydrocarbon ring with 5 to 7 carbon atoms;
Z is =CH-(alk)-M, =CH-(alk)-CO-(alk)-M, =N-(alk)-M,
   - (alk)-CH=CH-(alk)-M, =N-(alk)-SO₂M,
   - (alk)-SO₂M, -(alk)-N(R₃)-(alk)-M, -(alk)-CO-(alk)-M,
   - (alk)-N(R₃)-(alk)-CO-(alk)-M,
   - (alk)-CO-(alk)-N(R₃)-(alk)-M, -(alk)-N(R₃)-SO₂-(alk)-M,
   - (alk)-SO₂-N(R₃)-(alk)-M,
   - (alk)-N(R₃)-(alk)-N(R₃)-(alk)-M,
   - (alk)-N(R₃)-(alk)-N(R₃)-(alk)-CO-M, or -O-(alk)-CO-M,
      wherein (alk) is a chemical bond, an alkylene chain of the formula -(CᵢH₂ᵢ)-, or an alkylene chain of the formula -(CᵢH₂ᵢ₋₁)- which is substituted with a straight or branched alkyl group having 1 to 4 carbon atoms, wherein i is 0 to 6;
M is 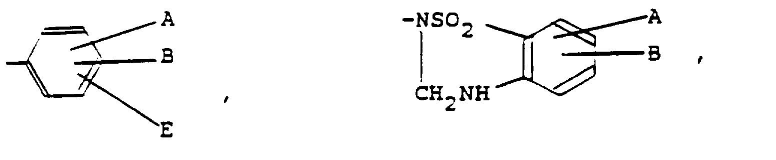 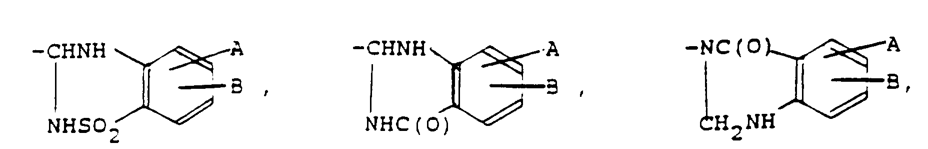 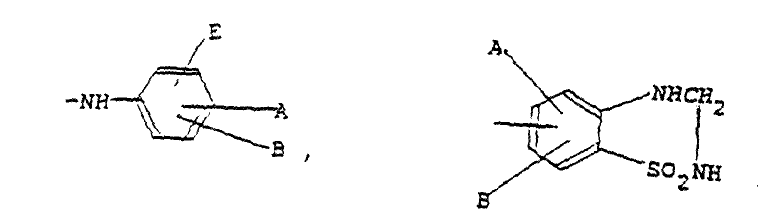 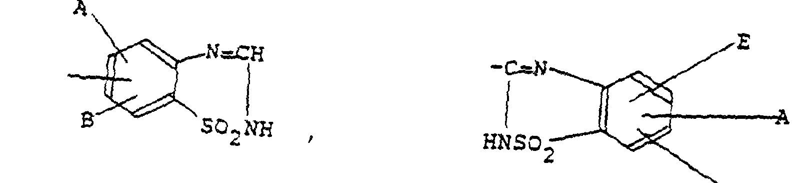 or 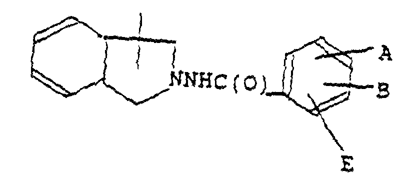 wherein A, B and E are independently H, C₁₋₆ alkyl, phenyl, benzyl, phenoxy, nitroalkylamino, alkanoylamino, alkanoylaminoalkyl, nitro, -OCH₂COOH, halogen, hydroxy, -CF₃, -SR, -OR, -NR₁R₂, -CO-NR₁R₂, -CO-Y₁, -SO₂R, -SO₂-NR₁R₂ or furfurylamino, provided that at least one of A and B is not hydrogen;
R, R₁, R₂ and R₃ each are independently hydrogen, alkyl having 1 to 8 carbon atoms, aryl having up to 12 carbon atoms, aryl-alkyl wherein the aryl moiety has up to 10 carbon atoms and the alkyl moiety has 1 to 6 carbon atoms, fused cycloalkylaryl having 8 to 12 carbon atoms, a heterocyclic group, or an alkyl group having 1 to 6 carbon atoms which is substituted with -NH₂, -NH-C(NH₂)=NH, or 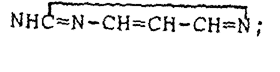 wherein R₁ and R₂ cannot be hydrogen;
   wherein the alkyl, cycloalkyl, aryl and fused arylcycloalkyl groups may carry substituents selected from the group consisting of alkoxy with 1 to 6 carbon atoms, -CF₃, -OH, -SH, halogen, -NO₂ and -COOR; provided that
   when the ring G is saturated or bicyclic and
Z is:
   - (alk)-N(R₃)-CO-phenyl
   - (alk)-N(R₃)-SO₂-phenyl
   - (alk)-N(R₃)-CH₂-phenyl 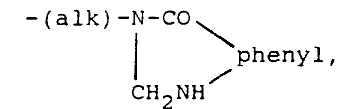 or 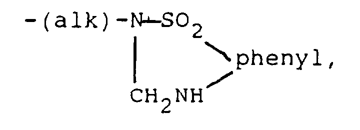 then (alk) must be branched alkyl and/or the phenyl moiety is substituted by amino, furfurylamino, -OCH₂CO₂H or two sulfamoyl groups.

Preferred substituents within the scope of the present invention include those wherein
Z is =CH-(CH₂)ᵢ-CO-M, -(CH₂)ᵢ-CO-M, -(CH₂)ᵢ-N(R₃)-CO-M,
- (CH₂)ᵢ-CO-N(R₃)-M, -(CH₂)ᵢ-N(R₃)-SO₂-M-(CH₂)ᵢM,
- (CH₂)ᵢ-SO₂M, -(CH₂)ᵢ-N(R₃)-M, -O-(CH₂)ᵢ-CO-M,
- N(R₃)-CH₂-(CH₂)ᵢ-N(R₃)-M, -O-(CH₂)ᵢ-M, =N-(CH₂)ᵢ-SO₂-M, =CH-(CH₂)ᵢ-M, or -CH=CHM, wherein M and R₃ are as defined above and i is 0 to 6 inclusive, provided that one carbon atom of a -(CH₂)ᵢ- linkage can be substituted with a straight or branchedchain alkyl group of up to 3 carbon atoms.

Y₁ and Y₂ are independently hydroxy, alkoxy containing up to 8 carbon atoms or phenyl - C₁₋₆ - alkoxy;
R₁ is H; alkyl having 1 to 8 carbon atoms; phenyl-alkyl wherein the alkyl has 1 to 4 carbon atoms, and more preferably phenethyl; or indanyl, e.g. 2-indanyl;
R₂ is H; alkyl having 1 to 8 carbon atoms; or an alkyl group having 1 to 8 carbon atoms, which is substituted with amino or an amino derivative such as -NH-C(NH₂)=NH, or 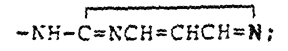 and R₂ is more preferably -CH₃ or NH₂(CH₂)₄-.

In the preferred embodiment, ring G is pyrrole, or a pyrrole ring containing one carbon-carbon double bond. In a preferred embodiment when G forms a fused ring system, X₁ and X₂ and T form tetrahydroquinoline or tetrahydroisoquinoline, or X₁ and X₂ form a pyrrole ring. The pyrrole ring of the one-ring or two-ring system G is preferably unsubstituted or substituted with an R₃ group which is preferably -OH or alkyl containing 1 to 6 carbon atoms.

When the chain connecting the moiety M to the ring includes a -(CH₂)ᵢ -linkage where i is non-zero, one of the carbon atoms or that linkage can be substituted with a straight-chained or branched alkyl group of up to 6 carbon atoms. Preferred connecting chains Z include those attached at the ring G by a double bond; -NH-; -NHSO₂-; -NHC(0)-; -CH₂NHC(0)-; -NHNHC(0)-; -N(CH₃)CH₂-; -OCH₂CH₂-; -CH₂CH₂-; -N(CH₃)CH₂CH₂-; and -NHC(0)CH₂-.

M is preferably 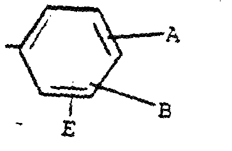 wherein A, B and E are as defined above.

A is preferably -NH₂; -OH; -OCH₂COOH; phenoxy; furfurylamino; alkoxy having up to 6 carbon atoms; or -SO₂NR₁R₂ wherein R₁ and R₂ are hydrogen, methyl, or C₂₋₃ alkyl, and more preferably -SO₂NH₂.

B is preferably halogen, and more preferably chloro; -CH₃; or -SO₂NR₁R₂ wherein R₁ and R₂ are hydrogen, methyl, or C₂₋₃ alkyl, and more preferably -SO₂NH₂; and E is preferably halogen or hydrogen.

Z is preferably
- (CH₂)₀₋₆CO-(CH₂)₁₋₆M,
- (CH₂)₀₋₆N(R₃)(CH₂)₁₋₆CO-(CH₂)₀₋₆M,
- (CH₂)₀₋₆N(R₃)-CO-(CH₂)₁₋₆M,
- (CH₂)₀₋₆CO-(CH₂)₁₋₆N(R₃)(CH₂)₀₋₆M,
- (CH₂)₀₋₆CO-N(R₃)(CH₂)₁₋₆M, -(CH₂)₀₋₆N(R₃)(CH₂)₁₋₆M,
- (CH₂)₀₋₆N(R₃)SO₂(CH₂)₁₋₆M, -(CH₂)₁₋₆O(CH₂)₀₋₆M,
- (CH₂)₀₋₆CH=CH(CH₂)₁₋₆M,
- (CH₂)₀₋₆SO₂-N(R₃)-(CH₂)₀₋₆M,
- (CH₂)₀₋₆N(R₃)-(CH₂)₀₋₆N(R₃)-(CH₂)₀₋₆)-CO-M,
- N(R₃)-(CH₂)₁₋₇N(R₃)(CH₂)₁₋₆M,
- CH₂)₁₋₆-N(R₃)(CH₂)₁₋₇N(R₃)(CH₂)₀₋₆M, or
- (CH₂)₀₋₆-N(R₃)-N(R₃)-(CH₂)₀₋₆M,
   wherein one carbon atom of a CH₂ group can be substituted with a straight or branched-chain alkyl group having 1 to 6 carbon atoms.

The alkyl groups in general include straight-chained and branched groups, including methyl, ethyl, propyl, isopropyl, butyl, isobutyl, tert-butyl, amyl, iso-amyl and hexyl. By "halogen" is meant chloro, bromo, iodo and fluoro.

Preferred substitutents for R₁ and/or R₂ also include cycloalkyl groups, aryl groups, heterocyclic groups, and fused aryl-cycloalkyl groups, as defined herein. The preferred cycloalkyl groups include cyclopropyl, cyclobutyl, cyclopentyl, cyclohexyl, cycloheptyl, adamantyl, or norbornyl. The preferred aryl and fused aryl-cycloalkyl groups include phenyl, indolyl, indolinyl, indanyl, naphthyl, tetrahydronaphthyl, and decahydronaphthyl. Preferred heterocyclic groups include pyridyl, quinolyl, isoquinolyl, tetrahydroquinolyl, tetrahydroisoquinolyl, decahydroquinolyl, decahydroisoquinolyl, pyrrolidyl, pyrrolyl, morpholinyl, furyl, tetrahydrofuryl, furfuryl, benzimidazolyl, thienyl, and imidazolyl. Preferred aryl-alkyl substituents include benzyl and phenethyl. Preferred substituents on the alkyl, cycloalkyl, aryl, and fused aryl-cycloalkyl substituents include alkyl and alkoxy with 1 to 6 carbon atoms, -CF₃, -OH, -NH₂, phenoxy, -NR₁R₂, -COOH, -CN, -SH, halogen, -NO₂, and COOR, particularly COO-C₁₋₆ alkyl.

Compounds according to formula (I) can contain asymmetric centers at the carbon atoms marked thus: C*. Each of these carbon atoms can have an (R) or an (S) configuration, and preferably (S). Individual optical diastereoisomers as well as mixtures thereof are considered to be within the scope of this invention. When diastereoisomeric products result from the synthetic procedures, the desired diastereoisomeric product can be separated by conventional chromatographic or fractional crystallization methods.

The compounds of formula (I) can be prepared by coupling compounds of formulas (II) and (III) 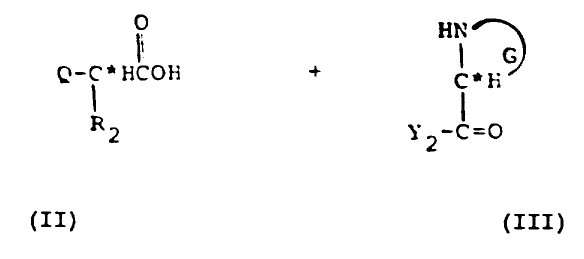 in which the substituents on ring G, including Z are previously present or are subsequently attached. The various substituents on compounds (II) and (III) have been defined above.

It will be recognized by those skilled in the art that the coupling of compounds (II) and (III) can be carried out by conventional peptide linkage techniques, e.g. in the presence of a coupling agent such as DCC (N,N' -dicyclohexylcarbodiimide) or CDI (N,N' -carbonyldiimidazole). Alternately, one may prefer to convert the -COOH group of compound (II) to -C(O)Cl, and then react the resulting intermediate with compound (III). Alternately one may preferably convert the compound (II) to the corresponding N-carboxyanhydride (NCA) by allowing (II) to react with phosgene, and then react the resulting N-carboxyanhydride with compound (III) to yield the desired intermediate. it will further be recognized that the nitrogen atom which is between the carbon atoms to which R₁ and R₂ are attached can be protected with a blocking group such as 2,2,2-trichloroethoxycarbonyl or benzyloxycarbonyl. The protecting group is subsequently removed, preferably after compounds (II) and (III) have been joined together. other nitrogen atoms, in substituents such as NH₂ (CH₂)₄, should be protected and then deprotected in a similar manner. Similarly, Y₁ and Y₂ are preferably converted to ethoxy, t-butoxy, or benzyloxy, before the intermediates are reacted. If the free acid is desired, it is subsequently obtained by removal of the esterifying group in a known manner.

The compounds of the present invention in which one of Y₁ and Y₂ is -OH and the other is alkoxy, such as methoxy or ethoxy, are preferably made by reacting compounds (II) and (III) as shown above in which one of Y₁ and Y₂ is the desired alkyl ester, and the other is an easily cleaved ester group such as t-butoxy. The amide intermediate thus prepared yields upon a mild acid hydrolysis the desired monoester-monoacids.

When Q contains sulfur, the preferred synthetic route is via the acid chloride. Alternately, compounds of formula (I) can be prepared by introducing by substitution or condensation reactions the substituent Z onto the remaining moiety of the formula (I) compounds: 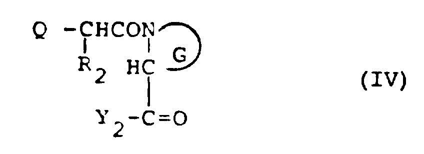 using suitable reactants to introduce the substituents represented by Z.

For example, 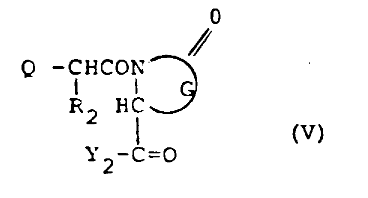 can be condensed with a hydrazine or amide of the formula HN (R₃) M(R₃)(alk)-M or HN(R₃)(alk)-M to form a hydrazone and imine, respectively and as desired, the condensation product reduced to the corresponding amine or hydrazinyl product of formula (I). Similar substitution or condensation reactions can be used to introduce other Z groups onto ring G as is known to those skilled in this art. Conventional reaction conditions are employed in such substitution and condensation reactions.

The compounds of this invention form salts with various inorganic and organic acids and bases which are also within the scope of the invention. Such salts include ammonium salts, alkali metal salts like sodium and potassium salts (which are preferred), alkaline earth metal salts like the calcium and magnesium salts, salts with organic bases e.g., dicyclohexylamine salts, N-methyl-D-glucamine and salts with amino acids like arginine or lysine. Also, salts with organic and inorganic acids may be prepared, e.g., HCl, HBr, H₂SO₄, H₃PO₄, as well as methanesulfonic, toluenesulfonic, maleic, acetic, malic, citric, fumaric and camphorsulfonic acids. The non-toxic physiologically acceptable salts are preferred, although other salts are also useful, e.g., in isolating or purifying the product.

The salts may be formed by conventional means, as by reacting the free acid or free base forms of the product with one or more equivalents of the appropriate base or acid in a solvent such as water which is then removed in vacuo or by freeze-drying, or by exchanging the cations of an existing salt for another cation on a suitable ion exchange resin.

The action of the enzyme renin on angiotensinogen, a pseudoglobuline in blood plasma, produces the decapeptide angiotensin I. Angiotensin I is converted by angiotensin converting enzyme (ACE) to the octapeptide angiotensin II. The latter is an active pressor substance which has been implicated as the causative agent in various forms of hypertension in various mammalian species, e.g., rats and dogs. The compounds within the scope of this invention which intervene in the renin -to- angiotensin I -to- angiotensin II sequence inhibit angiotensin I converting enzyme and therefore are useful in reducing or relieving hypertension.

Furthermore, the compounds within the scope of the present invention which possess diuretic activity promote relief from hypertension by promoting diuresis, and consequently have utility in treating congestive heart failure. Compounds within the scope of this invention can also simultaneously possess ACE inhibitory and diuretic activity, which is particularly unexpected in view of the fact that such simultaneous activity cannot be predicted from prior art compounds. Thus by the administration of a composition containing one or a combination of compounds of formula (I) or pharmaceutically-acceptable salts thereof, hypertension in the species of mammal suffering therefrom is alleviated. A single dose, or preferably two to four divided daily doses, provided on a basis of about 0.1 to 100mg per kg per day, preferably about 1 to 50mg per kg per day, is appropriate to reduce blood pressure. The substance is preferably administered orally, but a parenteral route such as subcutaneously, intramuscularly, intravenously or intraperitoneally can also be employed.

The compounds of this invention are also indicated for use in reducing intraocular pressure, e.g., for treating glaucoma. This effect is obtained by administering the amounts indicated above to a host in need of such treatment.

The compounds of the invention can be utilized by formulating one or more of them in compositions such as tablets, capsules or elixirs for oral administration or in sterile solutions or suspension for parenteral administration. About 10 to 500mg of a compound or mixture of compounds of formula (I) or physiologically acceptable salt (s) thereof is compounded with a physiologically acceptable vehicle, carrier, excipient, binder, preservative, stabilizer, or flavor, in a unit dosage form as called for by accepted pharmaceutical practice. The amount of active substance in these compositions or preparations is such that a suitable dosage in the range indicated is obtained.

Illustrative of the adjuvants which may be incorporated in tablets, capsules and the like are the following: a binder such as gum tragacanth, acacia, corn starch or gelatin; an excipient such as dicalcium phosphate; a disintegrating agent such as corn starch, potato starch or alginic acid ; a lubricant such as magnesium stearate; a sweetening agent such as sucrose, lactose or saccharin; a flavoring agent such as peppermint, oil of wintergreen or cherry. When the dosage unit form is a capsule, it may contain in addition to materials of the above type a liquid carrier such as a fatty oil. Various other materials may be present as coatings or to otherwise modify the physical form of the dosage unit. For instance, tablets may be coated with shellac, sugar or both. A syrup or elixir may contain the active compound, sucrose as a sweetening agent, methyl and propyl parabens as preservatives, a dye and a flavoring such as cherry or orange flavor.

Sterile compositions for injection can be formulated according to conventional pharmaceutical practice by dissolving or suspending the active substance in a vehicle such as water for injection, a naturally occurring vegetable oil like sesame oil, coconut oil, peanut oil or cottonseed oil, or a synthetic fatty vehicle like ethyl oleate. Buffers, preservatives and antioxidants can be incorporated as required.

Specific embodiments of the invention are illustrated in the following Examples.

### EXAMPLE 1

### N-[N-[(1S)-1-(Hydroxycarbonyl)-3-phenylpropyl]-L-alanyl]-4-(2,4-disulfamoyl-5-chloroanilino) proline

A mixture of N-[N-[(1S)-1-(ethoxycarbonyl)-3-phenyl -propyl]-N-(2,2,2-trichloroethoxycarbonyl)-L-alanyl]-L-prolin-4-one ethyl ester (2mmol), 4-amino-6-chloro-N-N'-bis[dimethyl-amino)methylene]-1,3-benzenedisulfonamide (3mmol), sodium cyanoborohydride (5mmol) and molecular sieves in absolute ethanol is stirred at room temperature for several days, filtered and the volatiles are removed in vacuo. The residue is purified by HPLC. The 2,2,2-trichloroethoxycarbonyl protecting group is removed with zinc and acetic acid and the esters and sulfonamide protecting groups are removed by reaction with alkali to provide the product.

### EXAMPLE 2

### N-[N-[(1S)-1-(Hydroxycarbonyl)-3-phenylpropyl]-L-alanyl)-4-(2,4-disulfamoyl-5-chlorophenylimino)proline

To a mixture of N-[N-[(1S)-1-(ethoxycarbonyl)-3-phenylpropyl[-N-(2,2,2-trichloroethoxycarbonyl)-L-alanyl]-L-prolin-4-one ethyl ester (20mmol), 4-amino-6-chloro-N,N'-bis [(dimethylamino)methylene]-1,3-benzenedisulfonamide (35mmol) and molecular sieves in absolute ethanol is added anhydrous HCl. The mixture is heated at reflux for 24 h, cooled to room temperature, filtered and the volatiles are removed in vacuo. The residue is purified by HPLC. The intermediate is treated with zinc and acetic acid in ethyl acetate, to remove the 2,2,2-trichloroethoxycarbonyl protecting group, and with alkali, to remove the sulfonamide protecting groups and hydrolyze the esters, thereby providing the product.

### EXAMPLE 3

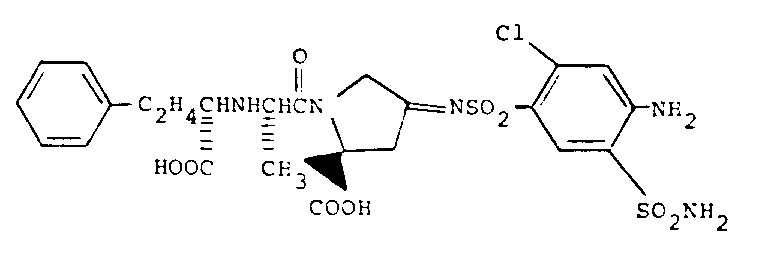

### N-[N-[(1S)-1-(Hydroxycarbonyl)-3-phenylpropyl]-L-alanyl]-4-(2-chloro-4-amino-5-sulfamoylphenyl-sulfonylimino)proline

To a mixture of N-[N-[(1S)-1-(ethoxycarbonyl)-3-phenylpropyl]-N-(2,2,2-trichloroethoxycarbonyl)-L-alanyl]-L-prolin-4-one ethyl ester (25mmol), 1-(N-(2,2,2-trichloroethoxycarbonyl)amino)-3-chloro-4,6-benzenedisulfonamide (55 mmol) and molecular sieves in absolute ethanol is added anhydrous HCl. The mixture is heated at reflux overnight, cooled to room temperature, filtered, and the volatiles are removed in vacuo. The residue is purified by HPLC. The 2,2,2-trichloroethoxycarbonyl protecting groups are removed with zinc and acetic acid in ethyl acetate and the esters are hydrolyzed with alkali to give the product.

### EXAMPLE 4

### N-[N-[(1S)-1-(Hydroxycarbonyl)-3-phenylpropyl]-L-alanyl]-3-(2,4-disulfamoyl-5-chloroanilino)-2,3-dihydroindole-2 -carboxylic acid

To a solution of N-[(1S)-1-(ethoxycarbonyl)-3-phenylpropyl]-N-(2,2,2-trichloroethoxycarbonyl)-L-alanine (5mmol) in methylene chloride at 0°C is added 1,1'-carbonyldiimidazole (5.5mmol). The mixture is stirred 30 min and 3-(2,4-disulfamoyl-5-chloroanilino)-2,3-dihydroindole-2-carboxylic acid ethyl ester (6mmol) is added. The mixture is warmed to room temperature, stirred overnight and the volatiles are removed in vacuo. Purification of the residue on HPLC provides the intermediate amide. The 2,2,2-trichloroethoxycarbonyl protecting group is removed with zinc and acetic acid and the esters are hydrolyzed with alkali to provide the product.

### EXAMPLE 5

### A. (4S)-1-((2S)-2-(N-((1S)-1-Ethoxycarbonyl-3-phenylpropyl)-N-(2,2,2-trichloroethoxycarbonyl)amino)propionyl)-4-(4 -chloro-2-(furfurylamino)-5-sulfonamidobenzoyl)aminoproline ethyl ester

A stirred solution of 11.3g (0.025mol) of N-((1S)-1-ethoxycarbonyl-3-phenylpropyl)-N-(2,2,2-trichloroethoxycarbonyl) -alanine in 200ml CH₂Cl₂ is treated with 5 drops DMF, followed by a solution of 10ml (0.12mol) of oxalyl chloride in 50ml CH₂Cl₂ added dropwise. The resulting solution is stirred for 1h after gas evolution ceases, then concentrated in vacuo. The residue is redissolved in 100ml CCl₄, decanted from a small amount of red oil, and concentrated again in vacuo.

A solution of 9.4g (0.020mol) of (4S)-4-(4-chloro -2-(furfurylamino)-5-sulfonamidobenzoyl)aminoproline ethyl ester and 2.5g (0.025mol) triethylamine in 200ml CH₂Cl₂ is stirred in an ice bath while a solution of the above acid chloride in 100ml CH₂Cl₂ is added dropwise. The mixture is allowed to reach room temperature overnight, then washed with dilute acid and dried. The solution is concentrated in vacuo and chromatographed on silica gel to afford the desired amide.

### B. (4S)-1-((2S)-2-(N-((1S)-1-Ethoxycarbonyl-3-phenylpropyl) amino)propionyl)-4-(4-chloro-2-(furfurylamino)-5-sulfonamidobenzoyl)aminoproline ethyl ester

To a solution of 9.4g (10.5mmol) of the above acylproline derivative in a mixture of 100ml EtOH and 50ml HOAc is added 6.5g (0.10mol) zinc dust. The resulting slurry is stirred at room temperature until TLC shows complete disappearance of starting material. Zinc salts and unreacted metal are filtered off and the filtrate concentrated in vacuo. Rapid chromatography on silica gel provides the pure amine.

### C. (4S)-1-((2S)-2-(N-((1S)-1-Hydroxycarbonyl-3-phenylpropyl)-amino)propionyl)-4-(4-chloro-2-(furfurylamino-5 -sulfonamidobenzoyl)aminoproline

To an ice-cooled solution of 3.5g (4.79mmol) of the above diester in 50ml EtOH is added dropwise 25ml 1N NaOH. The bath is removed and the solution stirred until TLC indicates complete ester hydrolysis. The solution is carefully neutralized with 1N HCl, then concentrated in vacuo and chromatographed on silica gel to give the desired product.

### EXAMPLE 6

### A. (4S)-1-((2S)-2-(N-((1S)-1-Ethoxycarbonyl-3-phenylpropyl)-N-(2,2,2-trichloroethoxycarbonyl)amino)propionyl)-4-(4 -amino-2-chloro-5-sulfonamidobenzenesulfonyl)aminoproline ethyl ester

A crude acid chloride is prepared from 5.22g (11.5mmol) of N-((1S)-1-ethoxycarbonyl-3-phenylpropyl)-N-(2,2,2-trichloroethoxycarbonyl)alanine as described in Example 7A. This is dissolved in 25ml CH₂Cl₂ and added dropwise to an ice-cooled solution of 4.3g (10.1mmol) of (4S)-4-(4-amino-2-chloro-5-sulfonamidobenzenesulfonyl)aminoproline ethyl ester and 1.5g (14.8mmol) of triethylamine in 150ml CH₂Cl₂. The reaction is stirred overnight at room temperature, then worked up as in Example 7A and chromatographed to give the title amide.

### B. (4S)-1-((2S)-2-(N-((1S)-1-Ethoxycarbonyl-3-phenylpropyl)-amino)propionyl)-4-(4-amino-2-chloro-5-sulfonamido benzenesulfonyl)aminoproline ethyl ester

A solution of 4.3g (5.0mmol) of product 8A in 50ml EtOH is treated sequentially with 25ml HOAc and 4.2g (66mmol) zinc dust. The reaction is stirred at room temperature until complete, as determined by TLC, then worked up as described in Example 7B to provide the desired amine.

### C. (4S)-1-((2S)-2-(N-((1S)-1-Hydroxycarbonyl-3-phenylpropyl) amino)-propionyl)-4-(4-amino-2-chloro-5-sulfonamidobenzenesulfonyl)aminoproline

A solution of 2.5g (3.6mmol) of product 8B in 35ml EtOH is hydrolyzed with 15ml 1N NaOH as described in Example 7C. Neutralization and chromatography of the crude product gives the pure diacid.

### EXAMPLE 7

### A. 1-((2S)-2-(N-((1S)-1-Ethoxycarbonyl-3-phenylpropyl)amino)-propionyl) -4-(2,3-dichloro-4-((ethoxycarbonyl)methoxy) -benzoyl)methyleneproline ethyl ester

The acid chloride of N-((1S)-1-ethoxycarbonyl-3-phenylpropyl)-N-(2,2,2-trichloroethoxycarbonyl)alanine (Example 7A) is dissolved in CH₂Cl₂ and added dropwise to a solution of 4-(2,3-dichloro-4-((ethoxycarbonyl)methoxy)benzoyl) methylene proline ethyl ester and triethylamine in CH₂Cl₂. Further reaction and purification as in example 7A gives the desired amide.

This is dissolved in EtOH and treated with HOAc and zinc dust. The reaction is stirred at room temperature until the starting material is consumed (TLC), then worked up as described for Example 7B. Careful chromatography on silica gel then gives the pure amine.

### B. 1-((2S)-2-(N-((1S)-1-Hydroxycarbonyl-3-phenyllpropyl) amino-propionyl)-4-(2,3-dichloro-4-(hydroxycarbonylmethoxy)-benzoyl)methyleneproline

A solution of the above triester (10A) in EtOH is cooled in ice and treated with 10% NaOH. The mixture is stirred until TLC shows complete conversion to triacid, then worked up and purified according to Example 7C to give the desired product.

### EXAMPLE 8

### 1-((2S)-2-(N-((1S)-1-Hydroxycarbonyl-3-phenylpropyl)amino)-propionyl-4-ethoxy-4-(5-chloro-2,4-disulfonamidophenyl) -aminoproline

4-Ethoxy-4-(5-chloro-2,4-disulfonamidophenyl)aminoproline ethyl ester is treated with the acid chloride of N-((1S)-1-ethoxycarbonyl-3-phenylpropyl)-N-(2,2,2-trichloroethoxycarbonyl)alanine as per Example 7A. Further deprotection (Example 7B) and hydrolysis (Example 7C) as previously described gives the desired product.

### EXAMPLE 9

### A. N-[N-(2,2,2-trichloroethoxycarbonyl)-N-[(1S)-1-(ethoxycarbonyl)-3-phenylpropyl]-L-alanyl]-4-[2-(3-sulfamoyl -4-chlorobenzoyl)hydrazin-1-yl]-L-proline ethyl ester

To a solution of N-[N-(2,2,2-trichloroethoxycarbonyl-N-[(1S)-1-(ethoxycarbonyl)-3-phenylpropyl]-L-alanyl]-L-prolin-4-one ethyl ester (3.90g, 6.57mmol) in 40ml ethanol (absolute) at 0-5°C and sodium tetrahydridoborate (0.744g, 19.7mmol) was added portionwise. The mixture was stirred 45 min at 0-5°C and water and ethyl acetate was added. The organic layer was washed with brine, dried (MgSO₄) and the volatiles were removed in vacuo. Purification of the residue on HPLC, using 25% hexanes in ethyl acetate as eluents, provided 2.82g of the solid product (12-A)N-[N-2,2,2-trichloroethoxycarbonyl)-N-[(1S)-1-(ethoxycarbonyl)-3-phenylpropyl] -L-alanyl]-4-[2-(3-sulfamoyl-4-chlorobenzoyl)hydrazin-L-proline ethyl ester.

### B. N-[N-[(1S)-1-(Hydroxycarbonyl)-3-phenylpropyl]-L-alanyl] -4-[2-(3-sulfamoyl-4-chlorobenzoyl]hydrazin-1-yl]-L-proline hydrochloride

To a solution of compound (12-A) (2.70g, 3.26mmol) in 20ml ethanol (absolute) and 10ml acetic acid was added zinc dust (2.12g, 32.6mmol). The mixture was stirred 3 h, filtered through Celite, diluted with ethyl acetate, washed with water and brine and dried (MgSO₄). Removal of the volatiles in vacuo provided a residue which was purified by HPLC using ethyl acetate as eluent. To the intermediate diester in 5ml ethanol at 0-5°C was added aqueous sodium hydroxide (10 molar equivalents of sodium hydroxide). The mixture was slowly warmed to room temperature and stirred overnight. The mixture was cooled with an ice bath, acidified to pH 1 with concentrated hydrochloric acid and extracted with ethyl acetate and ethanol (95:5). The organic layers were combined, washed with brine and dried (MgSO₄). Removal of the volatiles in vacuo provided a residue which was purified by trituration with acetonitrile to provide N-[N-[(1S)-1-(hydroxycarbonyl)-3-phenylpropyl]-L-alanyl]-4-[-2-(3-sulfamoyl-4-chlorobenzoyl)hydrazin-1-yl]-L-proline hydrochloride, m.p. 168°C (dec). This compound is a potent inhibitor of the angiotensin-converting enzyme.

### EXAMPLE 10

### A. (4RS)-N-(N-((1S)-1-Ethoxycarbonyl-3-phenylpropyl)-L-alanyl-4-(N-methyl-N-((6-chloro-7-sulfamyl-3,4-dihydro -2H-1,2,4-benzothiadizin-1,1-dioxide-3-yl)methyl) -amino)proline ethyl ester (13A)

To a solution of 3.7g (8.2mmol) of N-(N-((1S)-1-ethoxycarbonyl-3-phenylpropyl)-L-alanyl)-L-proline-4-one ethyl ester hydrochloride and 3.6g (9.5mmol) of 3-(methyl-amino)methyl-6-chloro-7-sulfamyl-3,4-dihydro-2H-1,2,4-benzothiadizine-1,1-dioxide hydrochloride in 25ml of DMF was added 4g MgSO₄. The mixture was stirred for 30 min at room temperature, then treated with a solution of 0.6g (0.01mol) sodium cyanoborohydride in 5ml DMF. After stirring overnight, the mixture was partitioned between water and EtOAc, the aqueous phase washed with EtOAc, then CH₂Cl₂ and the combined organics washed with H₂O and brine.

The combined organic extracts were stirred with 2.3g oxalic acid overnight and the resulting paste rinsed with dry EtOAc. The precipitate was redissolved in water, neutralized with excess K₂CO₃, and extracted with two portions of EtOAc. The extracts were concentrated in vacuo to give 1.7g (28%) of product (13-A) as a mixture of diastereomers, m.p. 79-85.

### B. (4RS)-N-(N-((1S)-1-Hydroxycarbonyl-3-phenyllpropyl)-L-alanyl)-4-((N-methyl-N-(6-chloro-7-sulfamyl-3,4-dihydro -2H-1,2,4-benzothiadiazine-1,1-dioxide-3-yl)methyl) -amino)proline (13-B)

The diester (13-A), (1.7g, 2.3mmol) was stirred in 10ml 50% EtOH and treated with 10ml 5% NaOH. After 2 h, TLC indicated complete hydrolysis. The mixture was acidified with 8ml 2N HCl. The resulting precipitate was washed with water, redissolved in 35ml 0.5 N HCl, washed with 10ml EtOAc, and lyophilized. The residue was chromatographed on silica gel to give 1.1g (72%) of compound (13-B), m.p. 193-197. This compound is a potent inhibitor of the angiotensin-converting enzyme.

### EXAMPLE 11

### (4R)-4-[5-Sulfamoyl)-4-chloro-2-[(2-furanylmethyl)amino]-benzoxy]-N-[N-[(1S)-1-(hydroxycarbonyl)-3-phenylpropyl]-L-alanyl]-L-proline

A mixture of furosemide (2.9g, 8.8mmol) and 1,1'-carbonyldiimidazole (1.5⁻g, 9.7mmol) in 30ml anhydrous tetrahydrofuran (THF) was stirred at 25°C for 1 h solution of (4R)-N-[N-[(1S)-1-(ethoxycarbonyl)-3-phenylpropyl]-L-alanyl]-4-hydroxy-L-proline ethyl ester (3.7g, 8.8mmol) in 40ml THF was added. The solution was heated at reflux for 20 h, cooled to room temperature and the volatiles were removed in vacuo. The residue was diluted with ethyl acetate, washed with water, aqueous HCl and brine and dried (MgSO₄). Removal of the volatiles in vacuo and purification of the residue by HPLC, using 50% ethyl acetate in hexane as eluents, provided the solid product. The ethyl esters can be hydrolyzed with alkali and the product purified on reverse phase silica gel using water in acetonitrile as eluents.

The following compounds within the scope of this invention have also been made following the procedures described herein, and have been found to be potent inhibitors of the angiotensin-converting enzyme:

EXAMPLE 12

N-[N-[(1S)-1-hydroxycarbonyl-3-phenylpropyl]-L-alanyl]-4-[((-6-chloro-2H-1,2,4-benzothiadiazin-1,1-dioxide-7-yl)sulfonyl) -amino)-L-proline

EXAMPLE 13

N-[N-[(1S)-1-hydroxycarbonyl-3-phenylpropyl]-L-alanyl]-4-[2-(6-chloro-7-sulfamoyl-3' 4-dihydro-2H-1,2,4-benzothiaziazin-1,1-dioxide-3-yl)ethoxy]proline

## Claims (Claims for the following Contracting State(s): BE, CH, LI, DE, FR, GB, IT, LU, NL, SE)

1. A compound of the formula (I) 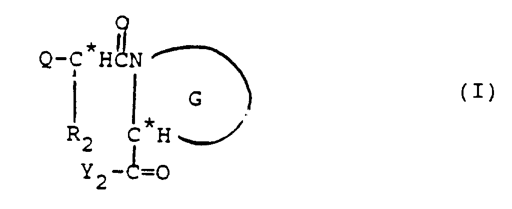 and pharmaceutically acceptable acid addition, alkali and alkaline earth metal salts thereof, wherein
Q is Y₁-CO-C*H(R₁)NH-, R₁-CO-S(C*H(R₁))₀₋₁, or HS-(C*HR₁)₀₋₁; wherein * indicates an asymmetric carbon; Y₁ and Y₂ are independently -OH, -OR, or -NR₁R₂;
G is 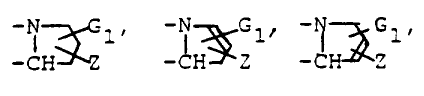 or 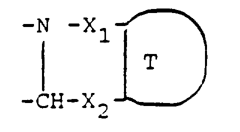
G₁ is H, -OH, C₁₋₆ alkyl or C₁₋₆ alkoxy;
X₁ and X₂ are independently a chemical bond or an alkylene bridge 1, 2 or 3 carbon atoms in length, provided that the ring which contains X₁ and X₂ contains 4 to 6 carbon atoms; one or both of X₁ and X₂ is optionally substituted with -OH, C₁₋₆ alkyl, or C₁₋₆ alkoxy; one of X₁ and X₂ is substituted with Z; X₁ and X₂ are otherwise substituted with hydrogen; and T is a saturated, unsaturated or aromatic hydrocarbon ring with 5 to 7 carbon atoms;
Z is =CH-(alk)-M, =CH-(alk)-CO-(alk)-M, =N-(alk)-M,
- (alk)-CH=CH-(alk)-M, =N-(ark)-SO₂M,
- (alk)-SO₂M, -(alk)-N(R₃)-(alk)-M, -(alk)-CO-(alk)-M,
- (alk)-N(R₃)-(alk)-CO-(alk)-M,
- (alk)-CO-(alk)-N(R₃)-(alk)-M, -(alk)-N(R₃)-SO₂-(alk)-M,
- (alk)-SO₂-N(R₃)-(alk)-M,
- (alk)-N(R₃)-(alk)-N(R₃)-(alk)-M,
- (alk)-N(R₃)-(alk)-N(R₃)-(alk)-CO-M, or -O-(alk)-CO-M,
wherein (alk) is a chemical bond, an alkylene chain of the formula -(CᵢH₂ᵢ)-, or an alkylene chain of the formula -(CᵢH₂ᵢ₋₁)- which is substituted with a straight or branched alkyl group having 1 to 4 carbon atoms, wherein i is 0 to 6;
M is 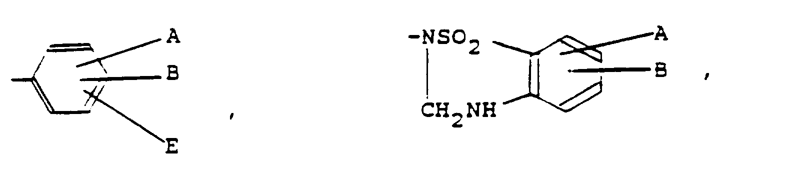 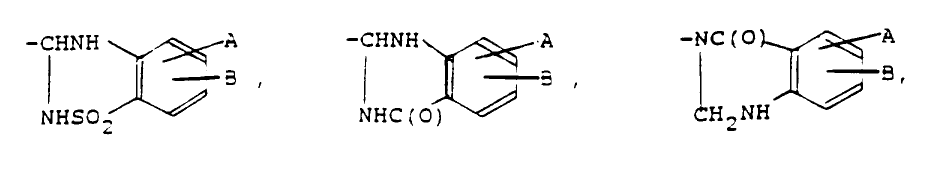 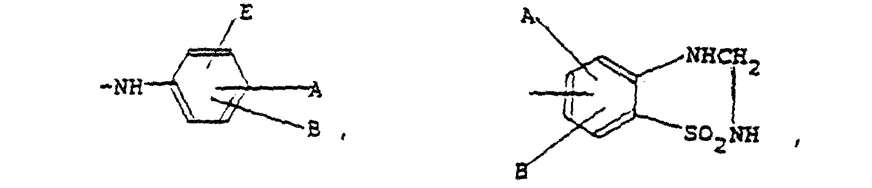 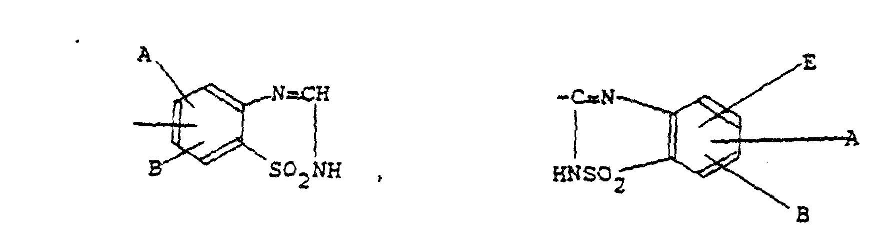 or 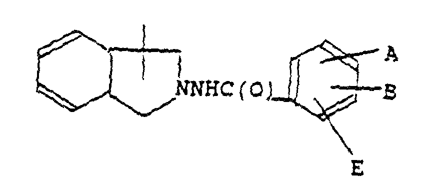 wherein A, B and E are independently H, C₁₋₆ alkyl, phenyl, benzyl, phenoxy, nitroalkylamino, alkanoylamino, alkanoylaminoalkyl, nitro, -OCH₂COOH, halogen, hydroxy, -CF₃, -SR,-OR,-NR₁R₂,-CO-NR₁R₂, -CO-Y₁, -SO₂R, -SO₂-NR₁R₂ or furfurylamino, provided that at least one of A and B is not hydrogen;
R, R₁, R₂ and R₃ each are independently hydrogen, alkyl having 1 to 8 carbon atoms, aryl having up to 12 carbon atoms, aryl-alkyl wherein the aryl moiety has up to 10 carbon atoms and the alkyl moiety has 1 to 6 carbon atoms, fused cycloalkylaryl having 8 to 12 carbon atoms, a heterocyclic group, or an alkyl group having 1 to 6 carbon atoms which is substituted with -NH₂, -NH-C(NH₂)=NH, or 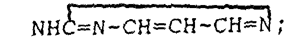 wherein R₁ and R₂ cannot be hydrogen;
wherein the alkyl, cycloalkyl, aryl and fused aryl-cycloalkyl groups may carry substituents selected from the group consisting of alkoxy with 1 to 6 carbon atoms, -CF₃, -OH, -SH, halogen, -NO₂ and -COOR;
provided that
(1) when the ring G is saturated or bicyclic and Z is:
- (alk)-N(R₃)-CO-phenyl
- (alk)-N(R₃)-SO₂-phenyl
- (alk)-N(R₃)-CH₂-phenyl 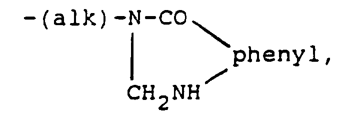 or 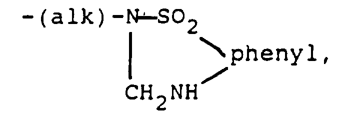 then (alk) must be branched alkyl and/or the phenyl moiety is substituted by amino, furfurylamino, -OCH₂CO₂H or two sulfamoyl groups.

2. The compound of claim 1 wherein
Z is =CH-(CH₂)ᵢCO-M, -(CH₂)ᵢ-CO-M, -(CH₂)ᵢ-N(R₃)-CO-M,
- (CH₂)ᵢ-CO-N(R₃)-M, -(CH₂)ᵢ-SO₂M, -(CH₂)ᵢ-N(R₃)-M,
- O-(CH₂)ᵢ-CO-M, -(CH₂)ᵢ-N(R₃)-SO₂-M-(CH₂)ᵢ-M,
- N(R₃)-CH₂-(CH₂)ᵢ-N(R₃)-M, -O-(CH₂)ᵢ-M, =N-(CH₂)ᵢ-SO₂-M, =N-(CH₂)ᵢ-M, =CH-(CH₂)ᵢ-M, or -CH=CHM,
wherein M, R₃ and i are as defined in claim 1 provided that one carbon atom of a -(CH₂)ᵢ-linkage can be substituted with a straight or branched-chain alkyl group of up to 3 carbon atoms.

3. The compound of claim 1 or 2 wherein Y₁ and Y₂ are independently -OH, alkoxy having 1 to 8 carbon atoms, or phenyl-C₁₋₆-alkoxy.

4. The compound of claim 3 wherein R₁ is H, alkyl or phenyl-alkyl.

5. The compound of claim 4 wherein R₂ is H, alkyl or aminoalkyl.

6. The compound of claim 5 wherein G is a pyrrole ring, tetrahydroquinoline or tetrahydroisoquinoline.

7. The compound of any of claims 1 to 6 wherein Z is
- (CH₂)₀₋₆CO-(CH₂)₁₋₆M,
- (CH₂)₀₋₆N(R₃)(CH₂)₁₋₆CO-(CH₂)₀₋₆M,
- (CH₂)₀₋₆N(R₃)-CO-(CH₂)₁₋₆M,
- (CH₂)₀₋₆CO-(CH₂)₁₋₆N(R₃)(CH₂)₀₋₆M,
- (CH₂)₀₋₆CO-N(R₃)(CH₂)₁₋₆M, -(CH₂)₀₋₆N(R₃)(CH₂)₁₋₆M,
- (CH₂)₀₋₆N(R₃)SO₂(CH₂)₁₋₆M, -(CH₂)₁₋₆O(CH₂)₀₋₆M,
- (CH₂)₀₋₆CH=CH(CH₂)₁₋₆M,
- (CH₂)₀₋₆SO₂-N(R₃)-(CH₂)₀₋₆M,
- (CH₂)₀₋₆N(R₃)-(CH₂)₀₋₆N(R₃)-(CH₂)₀₋₆)-CO-M,
- N(R₃)-(CH₂)₁₋₇N(R₃) (CH₂)₁₋₆M,
- CH₂)₁₋₆-N(R₃)(CH₂)₁₋₇N(R₃)(CH₂)₀₋₆M, or
- (CH₂)₀₋₆-N(R₃)-N(R₃)-(CH₂)₀₋₆M,
wherein one carbon atom of a CH₂ group can be substituted with a straight or branched-chain alkyl group having 1 to 4 carbon atoms.

8. The compound of claim 1 which is N-[N-(1S)-1-(hydroxycarbonyl)-3-phenylpropyl]-L-alanyl]-4-(2,4-disulfamoyl-5-chloroanilino)proline or N-[N-[(1S) -1-(hydroxy-carbonyl)-3-phenylpropyl]-L-alanyl]-4-(2,4- disulfamoyl-5-chlorophenylimino)proline or N-[N-[(1S)-1-(hydroxycarbonyl)-3-phenylpropyl]-L -alanyl]-4-(2-chloro-4-amino-5-sulfamoylphenylsulfonylimino)proline or N-[N-[(1S)-1-(hydroxycarbonyl)-3-phenylpropyl]-L-alanyl]-3-(2,4-disulfamoyl-5-chloroanilino)-2,3-dihydroindole-2-carboxylic acid or (4S)-1-((2S)-2-(N-(1S)-1-hydroxycarbonyl-3-phenylpropyl)amino)-propionyl)-4-(chloro-2-(furfurylamino)-5-sulfonamidobenzoyl) aminoproline or 1-((2S)-2-(N-((1S)-1-hydroxycarbonyl-3-phenyl-propyl)amino)-propionyl)-4-(2,3-dichloro-4-(carboxymethoxy)benzoyl) -methyleneproline or 1-((2S)-2-(N-((1S)-1-hydroxycarbonyl-3-phenylpropyl)amino)-propionyl-4-ethoxy-4-(5-chloro-2,4-disulfonamidophenyl)-aminoproline or N-[N-[(1S)-1-hydroxycarbonyl-3-phenylpropyl]-L-alanyl]-4-[2-(6-chloro-7-sulfamoyl-3,4dihydro-2H-1,2,4-benzothiadiazin-1,1 -dioxide-3-yl)ethoxy]proline or (4R)-4-[5-(sulfamoyl)-4-chloro-2-[(2-furanylmethyl)amino]-benzoxy]-N-[N-[(1S)-1-(hydroxycarbonyl)-3-phenylpropyl]-L-alanyl]-L-proline or (4R)-4-[5-(sulfamoyl)-4-chloro-2-[(2-furanylmethyl)amino]-benzoxy]-N-[N-(1S)-1-(ethoxycarbonyl)-3-phenylpropyl]-L-alanyl]-L-proline ethyl ester or N-[N-[(1S)-1-hydroxycarbonyl-3-phenylpropyl]-L-alanyl]-4-[((6-chloro-2-1,2,4-benzothiadiarin-1,1-dioxide-7-yl)sulfonyl)amino]-L-proline, or N-[N-[(1S)-1-hydroxycarbonyl-3-phenylpropyl]-L-alanyl]-4-[2-(3-sulfamoyl-4-chlorobenzoyl)hydrazin-1-yl]-L-proline, or N-[N-[(1S)-1-hydroxycarbonyl-3-phenylpropyl]-L-alanyl]-4-((N-methyl-N-(6-chloro-7-sulfamyl-3,4-dihydro-2H-1,2,4-benzothiadiazin-1,1-dioxide-3-yl)methyl) amino)proline, or N-[N-[(1S)-1-(Hydroxycarbonyl)-3-phenylpropyl] -L-alanyl]-3-(2,4-disulfamoyl-5-chloroanilino)-2,3-dihydroindole-2-carboxylic acid, or pharmaceutically acceptable acid addition, alkali and alkaline earth metal salt thereof.

9. A pharmaceutical preparation comprising a compound of any of claims 1 to 8 and a pharmaceutically acceptable carrier.

10. Use of the pharmaceutical preparation of claim 9 for the treatment of hypertension or glaucoma.

11. A process for preparing a compound of any of claims 1 to 8 comprising coupling a compound of formula (II) 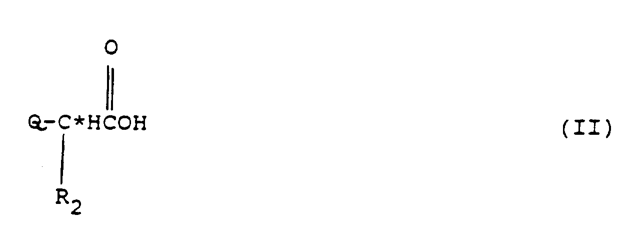 wherein the -COOH group has optionally been converted to a -COCl group or to the corresponding N-carboxyanhydride, with a compound of formula (III) 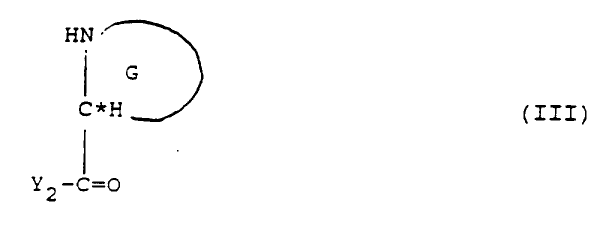 wherein Q, R₂, Y₂ and G are as defined in claim 1, and optionally forming salts of the compound of formula (I).

## Claims (Claims for the following Contracting State(s): AT)

1. A process for preparing a compound of the formula (I) 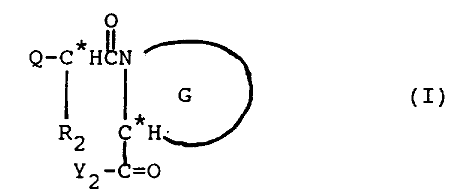 and pharmaceutically acceptable acid addition, alkali and alkaline earth metal salts thereof, wherein
Q is Y₁-CO-C*H(R₁)NH-, R₁-CO-S(C*H(R₁))₀₋₁, or HS-(C*HR₁)₀₋₁; wherein * indicates an asymmetric carbon; Y₁ and Y₂ are independently -OH, -OR, or -NR₁R₂;
G is 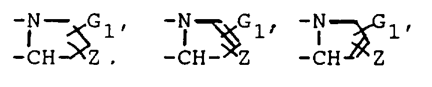 or 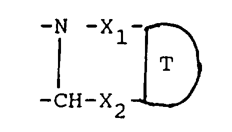
G₁ is H, -OH, C₁₋₆ alkyl or C₁₋₆ alkoxy;
X₁ and X₂ are independently a chemical bond or an alkylene bridge 1, 2 or 3 carbon atoms in length, provided that the ring which contains X₁ and X₂ contains 4 to 6 carbon atoms; one or both of X₁ and X₂ is optionally substituted with -OH, C₁₋₆ alkyl, or C₁₋₆ alkoxy; one of X₁ and X₂ is substituted with Z; X₁ and X₂ are otherwise substituted with hydrogen; and T is a saturated, unsaturated or aromatic hydrocarbon ring with 5 to 7 carbon atoms;
Z is =CH-(alk)-M, =CH-(alk)-CO-(alk)-M, =N-(alk)-M,
- (alk)-CH=CH-(alk)-M, =N-(alk)-SO₂M,
- (alk)-SO₂M, -(alk)-N(R₃)-(alk)-M, -(alk)-CO-(alk)-M,
- (alk)-N(R₃)-(alk)-CO-(alk)-M,
- (alk)-CO-(alk)-N(R₃)-(alk)-M, -(alk)-N(R₃)-SO₂-(alk)-M,
- (alk)-SO₂-N(R₃)-(alk)-M,
- (alk)-N(R₃)-(alk)-N(R₃)-(alk)-M,
- (alk)-N(R₃)-(alk)-N(R₃)-(alk)-CO-M, or -O-(alk)-CO-M,
wherein (alk) is a chemical bond, an alkylene chain of the formula -(CᵢH₂ᵢ)-, or an alkylene chain of the formula -(CᵢH₂ᵢ₋₁)- which is substituted with a straight or branched alkyl group having 1 to 4 carbon atoms, wherein i is 0 to 6;
M is 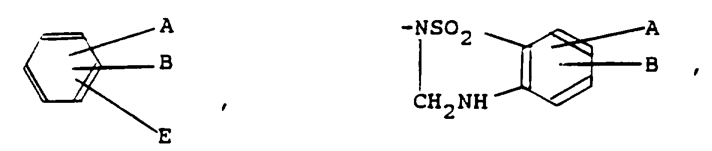 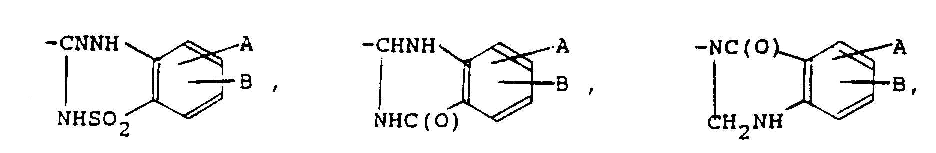 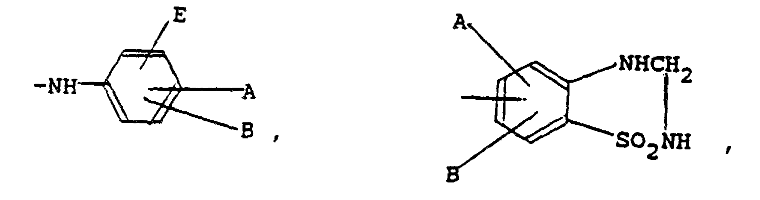 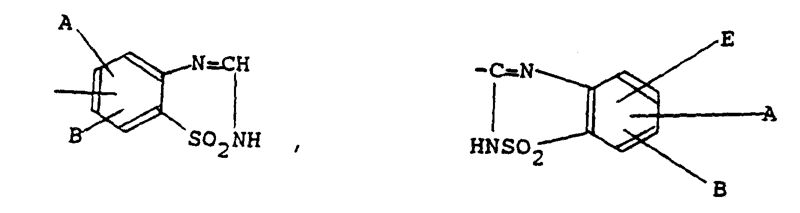 or 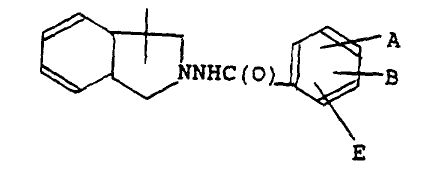 wherein A, B and E are independently H, C₁₋₆ alkyl, phenyl, benzyl, phenoxy, nitroalkylamino, alkanoylamino, alkanoylaminoalkyl, nitro, -OCH₂COOH, halogen, hydroxy, -CF₃, -SR, -OR, -NR₁R₂, -CO-NR₁R₂, -CO-Y₁, -SO₂R, -SO₂-NR₁R₂ or furfurylamino, provided that at least one of A and B is not hydrogen;
R, R₁, R₂ and R₃ each are independently hydrogen, alkyl having 1 to 8 carbon atoms, aryl having up to 12 carbon atoms, aryl-alkyl wherein the aryl moiety has up to 10 carbon atoms and the alkyl moiety has 1 to 6 carbon atoms, fused cycloalkylaryl having 8 to 12 carbon atoms, a heterocyclic group, or an alkyl group having 1 to 6 carbon atoms which is substituted with -NH₂, -NH-C(NH₂)=NH, or 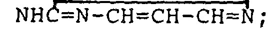 wherein R₁ and R₂ cannot be hydrogen;
wherein the alkyl, cycloalkyl, aryl and fused aryl-cycloalkyl groups may carry substituents selected from the group consisting of alkoxy with 1 to 6 carbon atoms, -CF₃, -OH, -SH, halogen, -NO₂ and -COOR;
provided that when the ring G is saturated or bicyclic and z is
- (alk)-N(R₃)-CO-phenyl
- (alk)-N(R₃)-SO₂-phenyl
- (alk)-N(R₃)-CH₂-phenyl 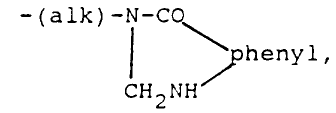 or 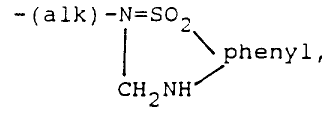 then (alk) must be branched alkyl and/or the phenyl moiety is substituted by amino, furfurylamino, -OCH₂CO₂H or two sulfamoyl groups.
comprising coupling a compound of formula (II) 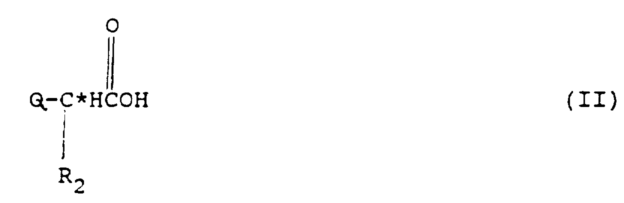
wherein the -COOH group has optionally been converted to a -COCl group or to the corresponding N-carboxyanhydride, with a compound of formula (III) 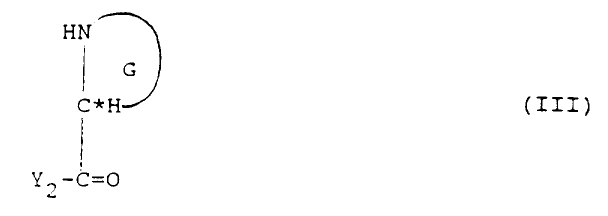
wherein Q, R₂, Y₂ and G are as defined above, and optionally forming salts of the compound of formula (I).

2. The process of claim 1 wherein
Z is =CH-(CH₂)ᵢCO-M, -(CH₂)ᵢ-CO-M, -(CH₂)ᵢ-N(R₃)-CO-M,
- (CH₂)ᵢ-CO-N(R₃)-M, -(CH₂)ᵢ-SO₂M, -(CH₂)ᵢ-N(R₃)-M,
- O-(CH₂)ᵢ-CO-M, -(CH₂)ᵢ-N(R₃)-SO₂-M-(CH₂)ᵢ-M,
- N(R₃)-CH₂-(CH₂)ᵢ-N(R₃)-M, -O-(CH₂)ᵢ-M, =N-(CH₂)ᵢ-SO₂-M, =N-(CH₂)ᵢ-M, =CH-(CH₂)ᵢ-M, or -CH=CHM,
wherein M and R₃ are as defined in claim 1 and i is 0 to 6 inclusive, provided that one carbon atom of a -(CH₂)ᵢ-linkage can be substituted with a straight or branched-chain alkyl group of up to 3 carbon atoms.

3. The process of claim 1 or 2 wherein Y₁ and Y₂ are independently -OH, alkoxy having 1 to 8 carbon atoms, or phenyl-C₁₋₆-alkoxy.

4. The process of claim 3 wherein R₁ is H, alkyl or phenyl-alkyl.

5. The process of claim 4 wherein R₂ is H, alkyl or aminoalkyl.

6. The process of claim 5 wherein G is a pyrrole ring, tetrahydroquinoline or tetrahydroisoquinoline.

7. The process of claim 6 wherein
M is 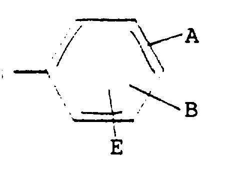 wherein A, B and E are as defined in claim 1.

8. The process of any of claims 1 to 7 wherein Z is
- (CH₂)₀₋₆CO-(CH₂)₁₋₆M,
- (CH₂)₀₋₆N(R₃)(CH₂)₁₋₆CO-(CH₂)₀₋₆M,
- (CH₂)₀₋₆N(R₃)-CO-(CH₂)₁₋₆M,
- (CH₂)₀₋₆CO-(CH₂)₁₋₆N(R₃)(CH₂)₀₋₆M,
- (CH₂)₀₋₆CO-N(R₃)(CH₂)₁₋₆M, -(CH₂)₀₋₆N(R₃)(CH₂)₁₋₆M,
- (CH₂)₀₋₆N(R₃)SO₂(CH₂)₁₋₆M, -(CH₂)₁₋₆O(CH₂)₀₋₆M,
- (CH₂)₀₋₆CH=CH(CH₂)₁₋₆M,
- (CH₂)₀₋₆SO₂-N(R₃)-(CH₂)₀₋₆M,
- (CH₂)₀₋₆N(R₃)-(CH₂)₀₋₆N(R₃)(CH₂)₀₋₆)-CO-M,
- N(R₃)-(CH₂)₁₋₇N(R₃)(CH₂)₁₋₆M,
- CH₂)₁₋₆-N(R₃)(CH₂)₁₋₇N(R₃)(CH₂)₀₋₆M, or
- (CH₂)₀₋₆-N(R₃)-N(R₃)-(CH₂)₀₋₆M,
wherein one carbon atom of a CH₂ group can be substituted with a straight or branched-chain alkyl group having 1 to 6 carbon atoms.

9. The process of claim 1 wherein the compound of formula (I) is N-[N-(1S)-1-(hydroxycarbonyl)-3-phenylpropyl]-L-alanyl]-4-(2,4-disulfamoyl-5-chloroanilino)proline or N-[N-[(1S) -1-(hydroxy-carbonyl)-3-phenylpropyl]-L-alanyl]-4-(2,4- disulfamoyl-5-chlorophenylimino)proline or N-[N-[(1S)-1-(hydroxycarbonyl)-3-phenylpropyl]-L-alanyl]-4-(2-chloro-4-amino-5-sulfamoyl-phenylsulfonylimino)proline or N-[N-[(1S)-1-(hydroxycarbonyl)-3-phenylpropyl]-L-alanyl]-3-(2,4-disulfamoyl-5-chloroanilino)-2,3-dihydroindole-2-carboxylic acid or (4S)-1-((2S)-2-(N-(1S)-1-hydroxycarbonyl-3-phenylpropyl)amino)-propionyl)-4-(chloro-2-(furfurylamino)-5-sulfonamidobenzoyl) aminoproline or 1-((2S)-2-[N-((1S)-1-hydroxycarbonyl-3-phenyl-propyl)amino)-propionyl)-4-(2,3-dichloro-4-(carboxymethoxy)benzoyl)-methyleneproline or 1-((2S)-2-(N-((1S)-1-hydroxycarbonyl-3-phenylpropyl)amino)-propionyl-4-ethoxy-4-(5-chloro-2,4-disulfonamidophenyl)-aminoproline or N-[N-[(1S)-1-hydroxycarbonyl-3-phenylpropyl]-L-alanyl]-4-[2-(6-chloro-7-sulfamoyl-3,4dihydro-2H-1,2,4-benzothiadiazin-1,1-dioxide-3-yl)ethoxy]proline or (4R)-4-[5-(sulfamoyl)-4-chloro-2-[(2-furanylmethyl)amino]-benzoxy]-N-[N-[(1S)-1-(hydroxycarbonyl)-3-phenylpropyl]-L-alanyl]-L-proline or (4R)-4-[5-(sulfamoyl)-4-chloro-2-[(2-furanylmethyl)amino]-benzoxy]-N-[N-(1S)-1-(ethoxycarbonyl)-3-phenylpropyl]-L-alanyl]-L-proline ethyl ester or N-[N-[(1S)-1-hydroxycarbonyl-3-phenylpropyl]-L-alanyl]-4-[((6-chloro-2-1,2,4-benzothiadiarin-1,1-dioxide-7-yl)sulfonyl)amino]-L-proline, or N-[N-[(1S)-1-hydroxycarbonyl-3-phenylpropyl]-L-alanyl]-4-[2-(3-sulfamoyl-4-chlorobenzoyl)hydrazin-1-yl]-L-proline, or N-[N-[(1S)-1-hydroxycarbonyl-3-phenylpropyl]-L-alanyl]-4-((N-methyl-N-(6-chloro-7-sulfamyl-3,4-dihydro-2H-1,2,4-benzothiadiazin-1,1-dioxide-3-yl)methyl)amino)proline, or N-[N-[(1S)-1-(Hydroxycarbonyl)-3-phenylpropyl]-L-alanyl]-3-(2,4-disulfamoyl-5-chloroanilino)-2,3-dihydroindole-2-carboxylic acid, or pharmaceutically acceptable acid addition, alkali and alkaline earth metal salt thereof.

10. A process for preparing an antihypertensive pharmaceutical preparation comprising formulating a compound or salt according to any of claims 1 to 9 and a pharmaceutically acceptable carrier.

11. A process for preparing an anti-glaucoma pharmaceutical preparation comprising formulating a compound or salt according to any of claims 1 to 9 and a pharmaceutically acceptable carrier.

## Patentansprüche (Patentansprüche für folgende(n) Vertragsstaat(en): BE, CH, LI, DE, FR, GB, IT, LU, NL, SE)

1. Verbindung der Formel (I) 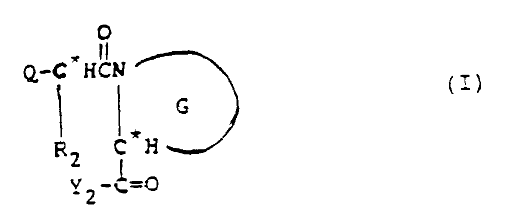 und pharmazeutisch annehmbare Säureadditions-, Alkali- und Erdalkalimetallsalze davon, wobei
Q Y₁-CO-C*H(R₁)NH-, R₁-CO-S(C*H(R₁))₀₋₁ oder HS-(C*HR₁)₀₋₁ ist; worin * ein asymmetrisches Kohlenstoffatom kennzeichnet;
Y₁ und Y₂ unabhängig voneinander -OH, -OR, oder -NR₁R₂ sind; 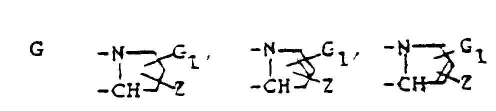 oder 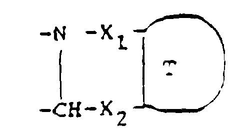 ist;
G₁ H, -OH, C₁₋₆-Alkyl oder C₁₋₆-Alkoxy ist;
X₁ und X₂ unabhängig voneinander eine chemische Bindung oder eine Alkylenbrücke mit einer Länge von 1, 2 oder 3 Kohlenstoffatomen darstellen, mit der Maßgabe, daß der Ring, der X₁ und X₂ enthält, 4 bis 6 Kohlenstoffatome enthält; eines oder beide von X₁ und X₂ gegebenenfalls mit-OH, C₁₋₆-Alkyl oder C₁₋₆-Alkoxy substituiert sind; eines von X₁ und X₂ mit Z substituiert ist; X₁ und X₂ ansonsten mit Wasserstoff substituiert sind;
und T ein gesättigter, ungesättigter oder aromatischer Kohlenwasserstoffring mit 5 bis 7 Kohlenstoffatomen ist;
Z =CH-(alk)-M, =CH-(alk)-CO-(alk)-M, =N-(alk)-M,
- (alk)-CH=CH-(alk)-M, =N-(alk)-SO₂M,
- (alk)-SO₂M, -(alk)-N(R₃)-(alk)-M, -(alk)-CO-(alk)-M,
- (alk)-N(R₃)-(alk)-CO-(alk)-M,
- (alk)-CO-(alk)-N(R₃)-(alk)-M, -(alk)-N(R₃)-SO₂-(alk)-M,
- (alk)-SO₂-N(R₃)-(alk)-M,
- (alk)-N(R₃)-(alk)-N(R₃)-(alk)-M,
- (alk)-N(R₃)-(alk)-N(R₃)-(alk)-CO-M oder -O-(alk)-CO-M ist,
worin (alk) eine chemische Bindung, eine Alkylenkette der Formel -(CᵢH₂ᵢ)- oder eine Alkylenkette der Formel -(CᵢH₂ᵢ₋₁)- ist, die mit einer geradkettigen oder verzweigten Alkylgruppen mit 1 bis 4 Kohlenstoffatomen substituiert ist, wobei i 0 bis 6 ist; 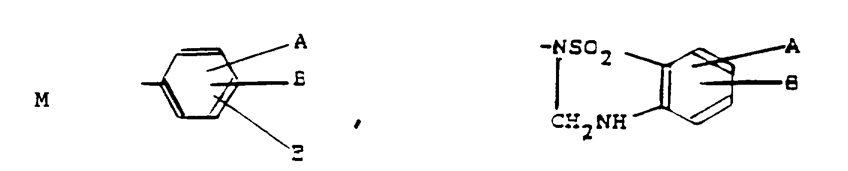 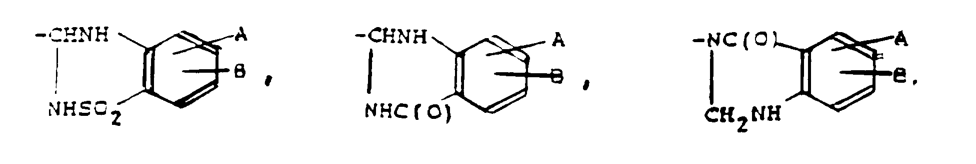 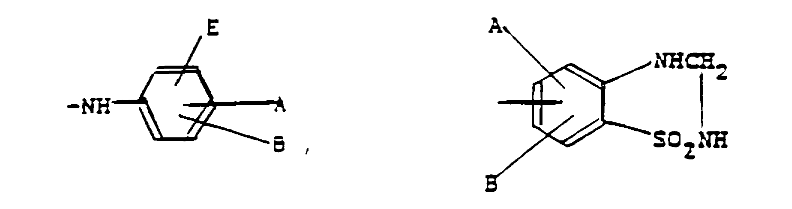 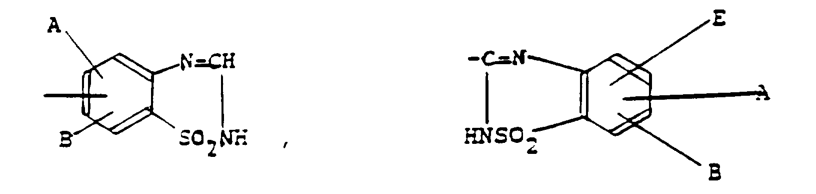 oder 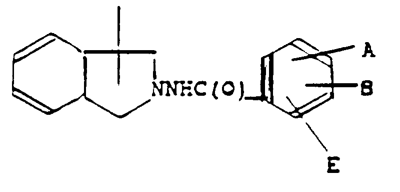 ist,
wobei A, B und E unabhängig voneinander H, C₁₋₆-Alkyl, Phenyl, Benzyl, Phenoxy, Nitroalkylamino, Alkanoylamino, Alkanoylaminoalkyl, Nitro, -OCH₂COOH, Halogen, Hydroxy, -CF₃, -SR, -OR, -NR₁R₂, -CO-NR₁R₂, -CO-Y₁, -SO₂R, -SO₂-NR₁R₂ oder Furfurylamino darstellen, mit der Maßgabe, daß mindestens eines von A und B nicht Wasserstoff ist;
R, R₁, R₂ und R₃ jeweils unabhängig voneinander Wasserstoff, Alkyl mit 1 bis 8 Kohlenstoffatomen, Aryl mit bis zu 12 Kohlenstoffatomen, Aryl-Alkyl, worin die Aryleinheit bis zu 10 Kohlenstoffatome und die Alkyleinheit 1 bis 6 Kohlenstoffatome aufweist, kondensiertes Cycloalkylaryl mit 8 bis 12 Kohlenstoffatomen, eine heterocyclische Gruppe oder eine Alkylgruppe mit 1 bis 6 Kohlenstoffatomen, die mit -NH₂, 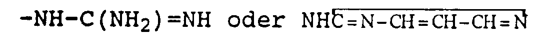 substituiert ist, darstellen, wobei R₁ und R₂ nicht Wasserstoff sein können;
wobei die Alkyl-, Cycloalkyl-, Aryl- und kondensierten Arylcycloalkylgruppen Substituenten, ausgewählt aus der Gruppe, bestehend aus Alkoxy mit 1 bis 6 Kohlenstoffatomen, -CF₃, -OH, -SH, Halogen, -NO₂ und -COOR, aufweisen können;
mit der Maßgabe, daß dann,
wenn der Ring G gesättigt oder bicyclisch ist, und Z:
- (alk)-N(R₃)-CO-phenyl
- (alk)-N(R₃)-SO₂-phenyl
- (alk)-N(R₃)-CH₂-phenyl 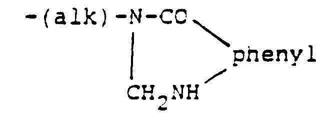 oder 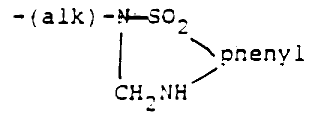 ist,
(alk) verzweigtes Alkyl sein muß und/oder die Phenyleinheit durch Amino, Furfurylamino, -OCH₂CO₂H oder zwei Sulfamoylgruppen substituiert ist.

2. Verbindung nach Anspruch 1, wobei
Z =CH-(CH₂)ᵢCO-M, -(CH₂)ᵢ-CO-M, -(CH₂)ᵢ-N(R₃)-CO-M,
- (CH₂)ᵢ-CO-N(R₃)-M, -(CH₂)ᵢ-SO₂M, -(CH₂)ᵢ-N(R₃)-M,
- O-(CH₂)ᵢ-CO-M, -(CH₂)ᵢ-N(R₃)-SO₂-M-(CH₂)ᵢ-M,
- N(R₃)-CH₂-(CH₂)ᵢ-N(R₃)-M, -O-(CH₂)ᵢ-M, =N-(CH₂)ᵢ-SO₂-M, =N-(CH₂)ᵢ-M, =CH-(CH₂)ᵢ-M oder -CH=CHM ist,
wobei M, R₃ und i wie in Anspruch 1 definiert sind, mit der Maßgabe, daß ein Kohlenstoffatom einer -(CH₂)ᵢ-Verbindung mit einer geradkettigen oder verzweigten Alkylgruppe von bis zu 3 Kohlenstoffatomen substituiert sein kann.

3. Verbindung nach Anspruch 1 oder 2, wobei Y₁ und Y₂ unabhängig voneinander -OH, Alkoxy mit 1 bis 8 Kohlenstoffatomen oder Phenyl-C₁₋₆-Alkoxy sind.

4. Verbindung nach Anspruch 3, wobei R₁ H, Alkyl oder Phenylalkyl ist.

5. Verbindung nach Anspruch 4, wobei R₂ H, Alkyl oder Aminoalkyl ist.

6. Verbindung nach Anspruch 5, wobei G ein Pyrrolring, Tetrahydrochinolin oder Tetrahydroisochinolin ist.

7. Verbindung nach einem der Ansprüche 1 bis 6, wobei Z
- (CH₂)₀₋₆CO-(CH₂)₁₋₆M,
- (CH₂)₀₋₆N(R₃)(CH₂)₁₋₆CO-(CH₂)₀₋₆M,
- (CH₂)₀₋₆N(R₃)-CO-(CH₂)₁₋₆M,
- (CH₂)₀₋₆CO-(CH₂)₁₋₆N(R₃)(CH₂)₀₋₆M,
- (CH₂)₀₋₆CO-N(R₃)(CH₂)₁₋₆M, -(CH₂)₀₋₆N(R₃)(CH₂)₁₋₆M,
- (CH₂)₀₋₆N(R₃)SO₂(CH₂)₁₋₆M, -(CH₂)₁₋₆O(CH₂)₀₋₆M,
- (CH₂)₀₋₆CH=CH(CH₂)₁₋₆M,
- (CH₂)₀₋₆SO₂-N(R₃)-(CH₂)₀₋₆M,
- (CH₂)₀₋₆N(R₃)-(CH₂)₀₋₆N(R₃)-(CH₂)₀₋₆)-CO-M,
- N(R₃)-(CH₂)₁₋₇N(R₃)(CH₂)₁₋₆M,
- (CH₂)₁₋₆-N(R₃)(CH₂)₁₋₇N(R₃)(CH₂)₀₋₆M oder
- (CH₂)₀₋₆-N(R₃)-N(R₃)-(CH₂)₀₋₆M ist,
wobei ein Kohlenstoffatom eine CH₂-Gruppe mit einer geradkettigen oder verzweigten Alkylgruppe mit 1 bis 4 Kohlenstoffatomen substituiert sein kann.

8. Verbindung nach Anspruch 1, die
N-[N-(1S)-1-(Hydroxycarbonyl)-3-phenylpropyl]-L-alanyl]-4-(2,4-disulfamoyl-5-chloranilino)prolin oder
N-[N-[(1S)-1-(Hydroxycarbonyl)-3-phenylpropyl]-L-alanyl]-4-(2,4-disulfamoyl-5-chlorphenylimino)prolin oder
N-[N-[(1S)-1-(Hydroxycarbonyl)-3-phenylpropyl]-L-alanyl]-4-(2-chlor-4-amino-5-sulfamoyl-phenylsulfonylimino)prolin oder
N-[N-[(1S)-1-(Hydroxycarbonyl)-3-phenylpropyl]-L-alanyl]-3-(2,4-disulfamoyl-5-chloranilino)-2,3-dihydroindol-2-carbonsäure oder
(4S)-1-((2S)-2-(N-(1S)-1-Hydroxycarbonyl-3-phenylpropyl)amino)-propionyl)-4-(chlor-2-(furfurylamino)-5-sulfonamidobenzoyl)aminoprolin oder
1-((2S)-2-(N-((1S)-1-Hydroxycarbonyl-3-phenylpropyl)amino)-propionyl)-4-(2,3-dichlor-4-(carboxymethoxy)benzoyl)-methylenprolin oder
1-((2S)-2-(N-((1S)-1-Hydroxycarbonyl-3-phenylpropyl)amino)-propionyl-4-ethoxy-4-(5-chlor-2,4-disulfonamidophenyl)-aminoprolin oder
N-[N-[(1S)-1-Hydroxycarbonyl-3-phenylpropyl]-L-alanyl]-4-[2-(6-chlor-7-sulfamoyl-3,4-dihydro-2H-1,2,4-benzothiadiazin-1,1-dioxid-3-yl)ethoxy]prolin oder
(4R)-4-[5-(Sulfamoyl)-4-chlor-2-[(2-furanylmethyl)amino]-benzoxy]-N-[N-[(1S)-1-(hydroxycarbonyl)-3-phenylpropyl]-L-alanyl]-L-prolin oder
(4R)-4-[5-(sulfamoyl)-4-chlor-2-[(2-furanylmethyl)amino]-benzoxy]-N-[N-[(1S)-1-(ethoxycarbonyl)-3-phenylpropyl]-L-alanyl]-L-prolinethylester oder
N-[N-[(1S)-1-Hydroxycarbonyl-3-phenylpropyl]-L-alanyl]-4-[((6-chlor-2-1,2,4-benzothiadiazin-1,1-dioxid-7-yl)sulfonyl)amino]-L-prolin oder
N-[N-[(1S)-1-Hydroxycarbonyl-3-phenylpropyl]-L-alanyl]-4-[2-(3-sulfamoyl-4-chlorbenzoyl)hydrazin-1-yl]-L-prolin oder
N-[N-[(1S)-1-Hydroxycarbonyl-3-phenylpropyl]-L-alanyl]-4-((N-methyl-N-(6-chlor-7-sulfamyl-3,4-dihydro-2H-1,2,4-benzothiadiazin-1,1-dioxid-3-yl)methyl)amino)prolin oder
N-[N-[(1S)-1-(Hydroxycarbonyl)-3-phenylpropyl]-L-alanyl]-3-(2,4-disulfamoyl-5-chloranilino)-2,3-dihydroindol-2-carbonsäure oder
pharmazeutisch annehmbare Säureadditions-, Alkali- und Erdalkalimetallsalze davon ist.

9. Pharmazeutisches Erzeugnis, umfassend eine Verbindung nach einem der Ansprüche 1 bis 8 und einen pharmazeutisch annehmbaren Träger.

10. Verwendung des pharmazeutischen Erzeugnisses nach Anspruch 9 für die Behandlung von Hypertonie und eines Glaukoms.

11. Verfahren zum Herstellen einer Verbindung nach einem der Ansprüche 1 bis 8, umfassend das Kuppeln einer Verbindung der Formel (II) 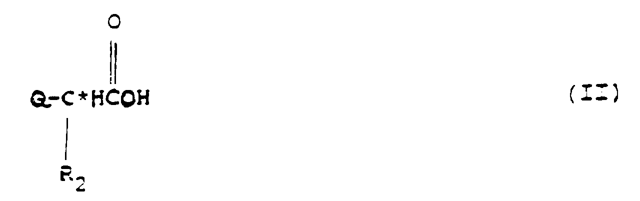 wobei die -COOH-Gruppe gegebenenfalls in eine -COCl-Gruppe oder das korrespondierende N-Carboxyanhydrid umgewandelt wurde,
an eine Verbindung der Formel (III) 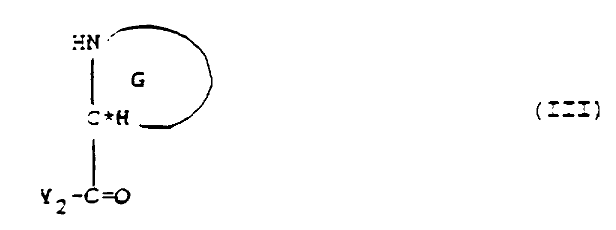 wobei Q, R₂, Y₂ und G wie in Anspruch 1 definiert sind, und gegebenenfalls das Bilden von Salzen der Verbindung der Formel (I).

## Patentansprüche (Patentansprüche für folgende(n) Vertragsstaat(en): AT)

1. Verfahren zum Herstellen einer Verbindung der Formel (I) 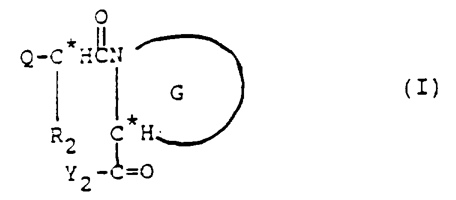 und pharmazeutisch annehmbarer Säureadditions-, Alkali- und Erdalkalimetallsalze davon, wobei
Q Y₁-CO-C*H(R₁)NH-, R₁-CO-S(C*H(R₁))₀₋₁ oder HS-(C*HR₁)₀₋₁ ist; worin * ein asymmetrisches Kohlenstoffatom kennzeichnet; Y₁ und Y₂ unabhängig voneinander -OH, -OR, oder -NR₁R₂ sind; 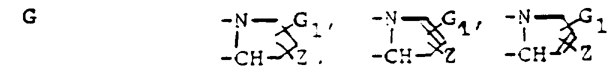 oder 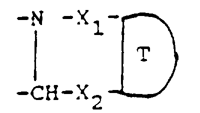 ist;
G₁ H, -OH, C₁₋₆-Alkyl oder C₁₋₆-Alkoxy ist;
X₁ und X₂ unabhängig voneinander eine chemische Bindung oder eine Alkylenbrücke mit einer Länge von 1, 2 oder 3 Kohlenstoffatomen darstellen, mit der Maßgabe, daß der Ring, der X₁ und X₂ enthält, 4 bis 6 Kohlenstoffatome enthält; eines oder beide von X₁ und X₂ gegebenenfalls mit -OH, C₁₋₆-Alkyl oder C₁₋₆-Alkoxy substituiert sind; eines von X₁ und X₂ mit Z substituiert ist;
X₁ und X₂ ansonsten mit Wasserstoff substituiert sind;
und T ein gesättigter, ungesättigter oder aromatischer Kohlenwasserstoffring mit 5 bis 7 Kohlenstoffatomen ist;
Z =CH-(alk)-M, =CH-(alk)-CO-(alk)-M, =N-(alk)-M,
- (alk)-CH=CH-(alk)-M, =N-(alk)-SO₂M,
- (alk)-SO₂M, -(alk)-N(R₃)-(alk)-M, -(alk)-CO-(alk)-M,
- (alk)-N(R₃)-(alk)-CO-(alk)-M,
- (alk)-CO-(alk)-N(R₃)-(alk)-M, -(alk)-N(R₃)-SO₂-(alk)-M,
- (alk)-SO₂-N(R₃)-(alk)-M,
- (alk)-N(R₃)-(alk)-N(R₃)-(alk)-M,
- (alk)-N(R₃)-(alk)-N(R₃)-(alk)-CO-M oder -O-(alk)-CO-M, ist,
worin (alk) eine chemische Bindung, eine Alkylenkette der Formel -(CᵢH₂ᵢ)- oder eine Alkylenkette der Formel -(CᵢH₂ᵢ₋₁)- ist die mit einer geradkettigen oder verzweigten Alkylgruppen mit 1 bis 4 Kohlenstoffatomen substituiert ist, wobei i 0 bis 6 ist; 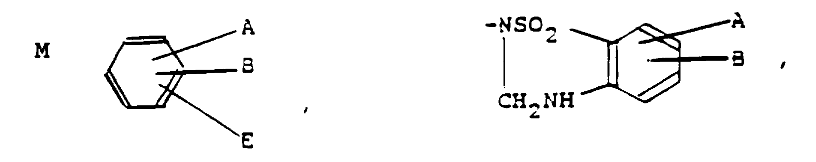 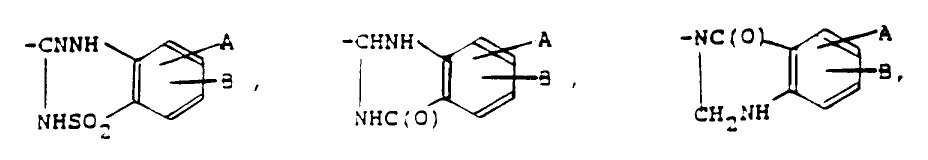 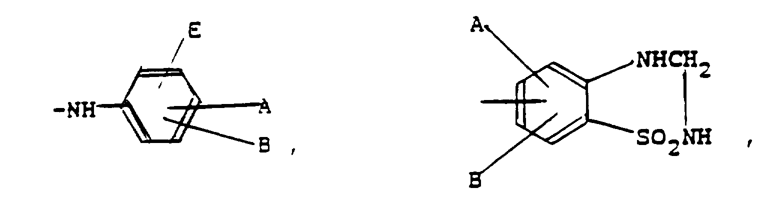 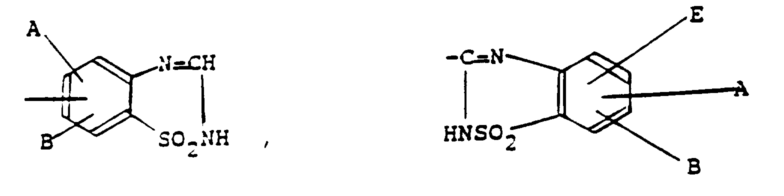
oder 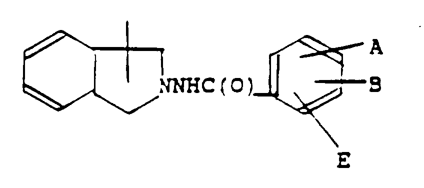 ist,
wobei A, B und E unabhängig voneinander H, C₁₋₆-Alkyl, Phenyl, Benzyl, Phenoxy, Nitroalkylamino, Alkanoylamino, Alkanoylaminoalkyl, Nitro, -OCH₂COOH, Halogen, Hydroxy,
-CF₃, -SR, -OR, -NR₁R₂, -CO-NR₁R₂, -CO-Y₁, -SO₂R, -SO₂-NR₁R₂ oder Furfurylamino darstellen, mit der Maßgabe, daß mindestens eines von A und B nicht Wasserstoff ist;
R, R₁, R₂ und R₃ jeweils unabhängig voneinander Wasserstoff, Alkyl mit 1 bis 8 Kohlenstoffatomen, Aryl mit bis zu 12 Kohlenstoffatomen, Aryl-Alkyl, worin die Aryleinheit bis zu 10 Kohlenstoffatome und die Alkyleinheit 1 bis 6 Kohlenstoffatome aufweist, kondensiertes Cycloalkylaryl mit 8 bis 12 Kohlenstoffatomen, eine heterocyclische Gruppe oder eine Alkylgruppe mit 1 bis 6 Kohlenstoffatomen, die mit -NH₂, 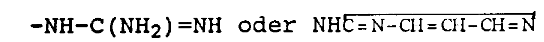 substituiert ist, darstellen, wobei R₁ und R₂ nicht Wasserstoff sein können;
wobei die Alkyl-, Cycloalkyl-, Aryl- und kondensierten Arylcycloalkylgruppen Substituenten, ausgewählt aus der Gruppe, bestehend aus Alkoxy mit 1 bis 6 Kohlenstoffatomen, -CF₃, -OH, -SH, Halogen, -NO₂ und -COOR, aufweisen können;
mit der Maßgabe, daß dann,
wenn der Ring G gesättigt oder bicyclisch ist, und Z:
- (alk)-N(R₃)-CO-phenyl
- (alk)-N(R₃)-SO₂-phenyl
- (alk)-N(R₃)-CH₂-phenyl 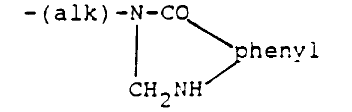 oder 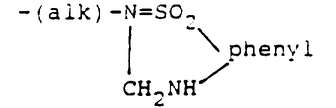 ist, (alk) verzweigtes Alkyl sein muß und/oder die Phenyleinheit durch Amino, Furfurylamino, -OCH₂CO₂H oder zwei Sulfamoylgruppen substituiert ist;
umfassend das Kuppeln einer Verbindung der Formel (II) 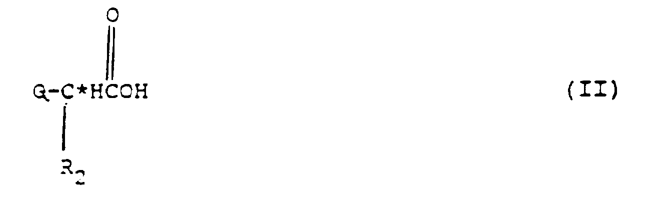
wobei die -COOH-Gruppe gegebenenfalls in eine -COCl-Gruppe oder das korrespondierende N-Carboxyanhydrid umgewandelt wurde, an eine Verbindung der Formel (III) 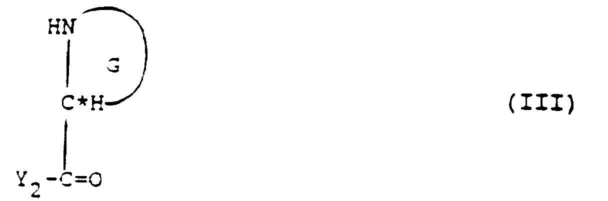 wobei Q, R₂, Y₂ und G wie vorstehend definiert sind, und gegebenenfalls das Bilden von Salzen der Verbindung der Formel (I) umfaßt.

2. Verfahren nach Anspruch 1, wobei
Z =CH-(CH₂)ᵢCO-M, -(CH₂)ᵢ-CO-M, -(CH₂)ᵢ-N(R₃)-CO-M,
- (CH₂)ᵢ-CO-N(R₃)-M, -(CH₂)ᵢ-SO₂M, -(CH₂)ᵢ-N(R₃)-M,
- O-(CH₂)ᵢ-CO-M, -(CH₂)ᵢ-N(R₃)-SO₂-M-(CH₂)ᵢ-M,
- N(R₃)-CH₂-(CH₂)ᵢ-N(R₃)-M, -O-(CH₂)ᵢ-M, =N-(CH₂)ᵢ-SO₂-M, =N-(CH₂)ᵢ-M, =CH-(CH₂)ᵢ-M oder -CH=CHM ist
wobei M und R₃ wie in Anspruch 1 definiert sind und i 0 bis einschließlich 6 ist, mit der Maßgabe, daß ein Kohlenstoffatom einer -(CH₂)ᵢ-Verbindung mit einer geradkettigen oder verzweigten Alkylgruppe mit bis zu 3 Kohlenstoffatomen substituiert sein kann.

3. Verfahren nach Anspruch 1 oder 2, wobei Y₁ und Y₂ unabhängig voneinander -OH, Alkoxy mit 1 bis 8 Kohlenstoffatomen oder Phenyl-C₁₋₆-Alkoxy sind.

4. Verfahren nach Anspruch 3, wobei R₁ H, Alkyl oder Phenylalkyl ist.

5. Verfahren nach Anspruch 4, wobei R₂ H, Alkyl oder Aminoalkyl ist.

6. Verfahren nach Anspruch 5, wobei G ein Pyrrolring, Tetrahydrochinolin oder Tetrahydroisochinolin ist.

7. Verfahren nach Anspruch 6, wobei 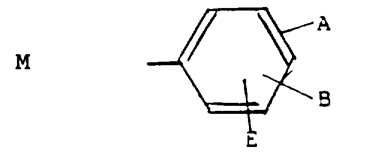
ist, worin A, B und E wie in Anspruch 1 definiert sind.

8. Verfahren nach einem der Ansprüche 1 bis 7, wobei Z
- (CH₂)₀₋₆CO-(CH₂)₁₋₆M,
- (CH₂)₀₋₆N(R₃)(CH₂)₁₋₆CO-(CH₂)₀₋₆M,
- (CH₂)₀₋₆N(R₃)-CO-(CH₂)₁₋₆M,
- (CH₂)₀₋₆CO-(CH₂)₁₋₆N(R₃)(CH₂)₀₋₆M,
- (CH₂)₀₋₆CO-N(R₃)(CH₂)₁₋₆M, -(CH₂)₀₋₆N(R₃)(CH₂)₁₋₆M,
- (CH₂)₀₋₆N(R₃)SO₂(CH₂)₁₋₆M, -(CH₂)₁₋₆O(CH₂)₀₋₆M,
- (CH₂)₀₋₆CH=CH(CH₂)₁₋₆M,
- (CH₂)₀₋₆SO₂-N(R₃)-(CH₂)₀₋₆M,
- (CH₂)₀₋₆N(R₃)-(CH₂)₀₋₆N(R₃)-(CH₂)₀₋₆)CO-M,
- N(R₃)-(CH₂)₁₋₇N(R₃)(CH₂)₁₋₆M,
- (CH₂)₁₋₆N(R₃)(CH₂)₁₋₇N(R₃)(CH₂)₀₋₆M oder
- (CH₂)₀₋₆-N(R₃)-N(R₃)-(CH₂)₀₋₆M ist,
wobei ein Kohlenstoffatom einer CH₂-Gruppe mit einer geradkettigen oder verzweigten Alkylgruppe mit 1 bis 6 Kohlenstoffatomen substituiert sein kann.

9. Verfahren nach Anspruch 1, wobei die Verbindung der Formel (I)
N-[N-[(1S)-1-(Hydroxycarbonyl)-3-phenylpropyl]-L-alanyl]-4-(2,4-disulfamoyl-5-chloranilino)prolin oder
N-[N-[(1S)-1-(Hydroxycarbonyl)-3-phenylpropyl]-L-alanyl]-4-(2,4-disulfamoyl-5-chlorphenylimino)prolin oder
N-[N-[(1S)-1-(Hydroxycarbonyl)-3-phenylpropyl]-L-alanyl]-4-(2-chlor-4-amino-5-sulfamoyl-phenylsulfonylimino)prolin oder
N-[N-[(1S)-1-(Hydroxycarbonyl)-3-phenylpropyl]-L-alanyl]-3-(2,4-disulfamoyl-5-chloranilino)-2,3-dihydroindol-2-carbonsäure oder
(4S)-1-((2S)-2-(N-(1S)-1-Hydroxycarbonyl-3-phenylpropyl)amino)-propionyl)-4-(chlor-2-(furfurylamino)-5-sulfonamidobenzoyl)-aminoprolin oder
1-((2S)-2-(N-((1S)-1-Hydroxycarbonyl-3-phenylpropyl)amino)-propionyl)-4-(2,3-dichlor-4-(carboxymethoxy)benzoyl)-methylenprolin oder
1-((2S)-2-(N-((1S)-1-Hydroxycarbonyl-3-phenylpropyl)amino)-propionyl-4-ethoxy-4-(5-chlor-2,4-disulfonamidophenyl)-aminoprolin oder
N-[N-[(1S)-1-Hydroxycarbonyl-3-phenylpropyl]-L-alanyl]-4-[2-(6-chlor-7-sulfamoyl-3,4-dihydro-2H-1,2,4-benzothiadiazin-1,1-dioxid-3-yl)ethoxy]prolin oder
(4R)-4-[5-(Sulfamoyl)-4-chlor-2-[(2-furanylmethyl)amino]-benzoxy]-N-[N-[(1S)-1-(hydroxycarbonyl)-3-phenylpropyl]-L-alanyl]-L-prolin oder
(4R)-4-[5-(Sulfamoyl)-4-chlor-2-[(2-furanylmethyl)amino]-benzoxy]-N-[N-[(1S)-1-(ethoxycarbonyl)-3-phenylpropyl]-L-alanyl]-L-prolinethylester oder
N-[N-[(1S)-1-Hydroxycarbonyl-3-phenylpropyl]-L-alanyl]-4-[((6-chlor-2-1,2,4-benzothiadiazin-1,1-dioxid-7-yl)sulfonyl)amino]-L-prolin oder
N-[N-[(1S)-1-Hydroxycarbonyl-3-phenylpropyl]-L-alanyl]-4-[2-(3-sulfamoyl-4-chlorbenzoyl)hydrazin-1-yl]-L-prolin oder
N-[N-[(1S)-1-Hydroxycarbonyl-3-phenylpropyl]-L-alanyl]-4-((N-methyl-N-(6-chlor-7-sulfamyl-3,4-dihydro-2H-1,2,4-benzothiadiazin-1,1-dioxid-3-yl)methyl)amino)prolin oder
N-[N-[(1S)-1-(Hydroxycarbonyl)-3-phenylpropyl]-L-alanyl]-3-(2,4-disulfamoyl-5-chloranilino)-2,3-dihydroindol-2-carbonsäure oder pharmazeutisch annehmbare Säureadditions-, Alkali- und Erdalkalimetallsalze davon ist.

10. Verfahren zum Herstellen eines antihypertonischen pharmazeutischen Erzeugnisses, umfassend das Formulieren einer Verbindung oder Salzes, hergestellt gemäß einem der Ansprüche 1 bis 9, und eines pharmazeutisch annehmbaren Trägers.

11. Verfahren zum Herstellen eines pharmazeutischen Antiglaukomieerzeugnisses, umfassend das Formulieren einer Verbindung oder Salzes, hergestellt nach einem der Ansprüche 1 bis 9, und eines pharmazeutisch annehmbaren Trägers.

## Revendications (Revendications pour l'(les) Etat(s) contractant(s) suivant(s): BE, CH, LI, DE, FR, GB, IT, LU, NL, SE)

1. Composé de formule (I) : 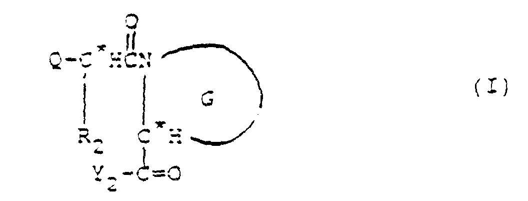 et ses sels, acceptables d'un point de vue pharmaceutique, d'addition avec un acide, de métaux alcalins et alcalino-terreux, où :
Q est Y₁-CO-C*H(R₁)NH-, R₁-CO-S(C*H(R₁))₀₋₁, ou HS-(C*HR₁)₀₋₁ ; où * désigne un carbone asymétrique ; Y₁ et Y₂, indépendamment l'un de l'autre, sont chacun -OH, -OR, ou -NR₁R₂ ;
G est 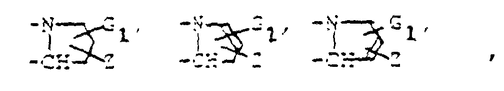 ou 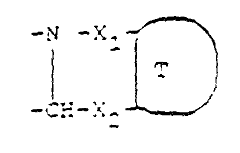
G₁ est H, -OH ou un radical alkyle en C₁₋₆ ou alcoxy en C₁₋₆ ;
X₁ et X₂, indépendamment l'un de l'autre, représentent chacun une liaison chimique ou un pont alkylène ayant 1, 2 ou 3 atomes de carbone, à condition que le noyau contenant X₁ et X₂ contienne de 4 à 6 atomes de carbone ; l'un des radicaux X₁ et X₂, ou les deux, sont éventuellement substitués par -OH, ou un radical alkyle en C₁₋₆ ou alcoxy en C₁₋₆ ; l'un des radicaux X₁ et X₂ est substitué par Z ; X₁ et X₂ sont autrement substitués par un hydrogène ; et T est un noyau hydrocarboné saturé, insaturé ou aromatique, ayant de 5 à 7 atomes de carbone ;
Z est =CH-(alk)-M, =CH-(alk)-CO-(alk)-M, =N-(alk)-M,
- (alk)-CH=CH-(alk)-M, =N-(alk)-SO₂M,
- (alk)-SO₂M, -(alk)-N(R₃)-(alk)-M, -(alk)-CO-(alk)-M,
- (alk)-N(R₃)-(alk)-CO-(alk)-M,
- (alk)-CO-(alk)-N(R₃)-(alk)-M, -(alk)-N(R₃)-SO₂-(alk)-M,
- (alk)-SO₂-N(R₃)-(alk)-M,
- (alk)-N(R₃)-(alk)-N(R₃)-(alk)-M,
- (alk)-N(R₃)-(alk)-N(R₃)-(alk)-CO-M, ou -O-(alk)-CO-M,
où (alk) est une liaison chimique, une chaîne alkylène de formule -(CᵢH₂ᵢ)-, ou encore une chaîne alkylène de formule -(C₁H₂ᵢ₋₁)-, qui est substituée par un groupe alkyle à chaîne droite ou ramifiée, ayant de 1 à 4 atomes de carbone, où i vaut de 0 à 6 ;
M est 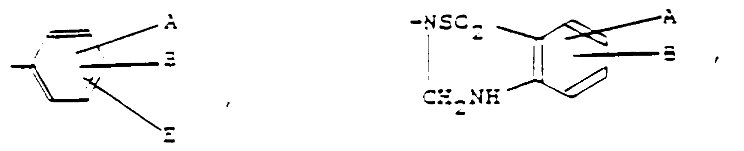 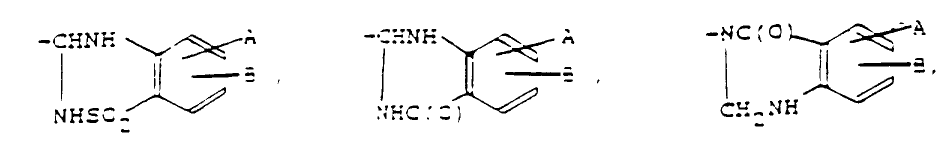 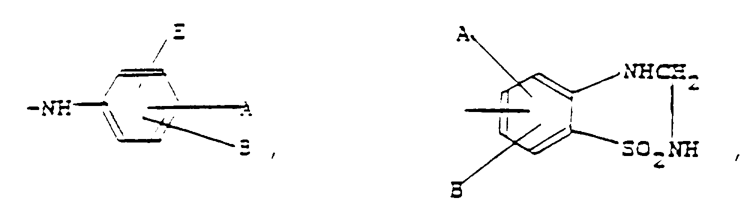 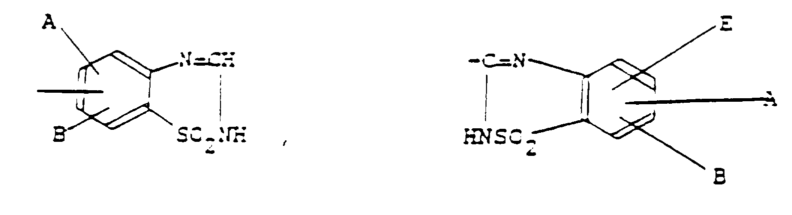 ou 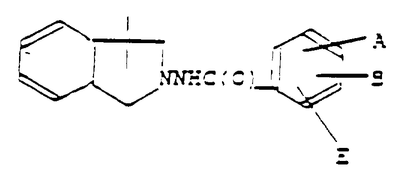
où A, B et E sont, indépendamment les uns des autres, H, un radical alkyle en C₁₋₆, phényle, benzyle, phénoxy, nitroalkylamino, alcanoylamino, alcanoylaminoalkyle, nitro, -OCH₂COOH, halogèno, hydroxy, -CF₃, -SR, -OR, -NR₁R₂, -CO-NR₁R₂, -CO-Y₁, -SO₂R, -SO₂-NR₁R₂ ou furfurylamino, du moment qu'au moins l'un des radicaux A et B n'est pas un hydrogène;
R, R₁, R₂ et R₃, chacun indépendamment des autres, est un hydrogène ou un radical alkyle ayant de 1 à 8 atomes de carbone, aryle ayant jusqu'à 12 atomes de carbone, arylalkyle dans lequel le fragment aryle a jusqu'à 10 atomes de carbone et le fragment alkyle a de 1 à 6 atomes de carbone, cycloalkylaryle condensé ayant de 8 à 12 atomes de carbone, un groupe hétérocyclique, ou encore un groupe alkyle ayant de 1 à 6 atomes de carbone, qui est substitué par -NH2, -NH-C(NH₂)=NH, ou 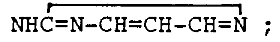 où R₁ et R₂ ne peuvent être des hydrogènes ;
où les groupes alkyle, cycloalkyle, aryle et arylcycloalkyle condensé peuvent porter des substituants choisis parmi l'ensemble comprenant les groupes alcoxy ayant de 1 à 6 atomes de carbone, -CF₃, -OH, -SH, halogèno, -NO₂ et -COOR ;
à condition que, quand le noyau G est saturé ou bicyclique, et Z est
- (alk)-N(R₃)-CO-phényl
- (alk)-N(R₃)-SO₂-phényl
- (alk)-N(R₃)-CH₂-phényl 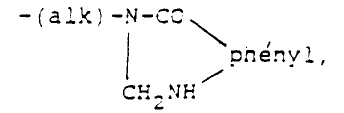 ou 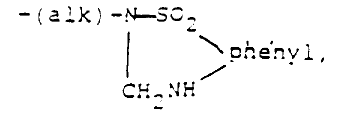 alors (alk) est un radical alkyle ramifié et/ou le fragment phényle est substitué par un substituant amino, furfurylamino, -OCH₂CO₂H ou deux groupes sulfamoyle.

2. Composé selon la revendication 1, dans lequel
Z est =CH-(CH₂)ᵢCO-M, -(CH₂)ᵢ-CO-M, -(CH₂)ᵢ-N(R₃)-CO-M,
- (CH₂)ᵢ-CO-N(R₃)-M, -(CH₂)ᵢ-SO₂M, -(CH₂)ᵢ-N(R₃)-M,
- O-(CH₂)ᵢ-CO-M, -(CH₂)ᵢ-N(R₃)-SO₂-M-(CH₂)ᵢ-M,
- N(R₃)-CH₂-(CH₂)ᵢ-N(R₃)-M, -O-(CH₂)ᵢ-M, =N-(CH₂)ᵢ-SO₂-M, =N-(CH₂)ᵢ-M, =CH-(CH₂)ᵢ-M, ou =CH=CHM, où M, R₃ et i
sont tels que définis dans la revendication 1, à condition qu'un atome de carbone d'un maillon -(CH₂)ᵢ- peut être substitué par un groupe alkyle à chaîne droite ou ramifiée, ayant jusqu'à 3 atomes de carbone.

3. Composé selon la revendication 1 ou 2, dans lequel Y₁ et Y₂, indépendamment l'un de l'autre, sont chacun -OH ou un radical alcoxy ayant de 1 à 8 atomes de carbone ou phényl-(alcoxy en C₁₋₆).

4. Composé selon la revendication 3, dans lequel R₁ est H ou un radical alkyle ou phénylalkyle.

5. Composé selon la revendication 4, dans lequel R₂ est H ou un radical alkyle ou aminoalkyle.

6. Composé selon la revendication 5, dans lequel G est un noyau pyrrole, tétrahydroquinoléine ou tétrahydroisoquinoléine.

7. Composé selon l'une quelconque des revendications 1 à 6, dans lequel Z est
- (CH₂)₀₋₆CO-(CH₂)₁₋₆M,
- (CH₂)₀₋₆N(R₃)(CH₂)₁₋₆CO-(CH₂)₀₋₆M,
- (CH₂)₀₋₆N(R₃)-CO-(CH₂)₁₋₆M,
- (CH₂)₀₋₆CO-(CH₂)₁₋₆N(R₃)(CH₂)₀₋₆M,
- (CH₂)₀₋₆CO-N(R₃)(CH₂)₁₋₆M, -(CH₂)₀₋₆N(R₃)(CH₂)₁₋₆M,
- (CH₂)₀₋₆N(R₃)SO₂(CH₂)₁₋₆M, -(CH₂)₁₋₆O(CH₂)₀₋₆M,
- (CH₂)₀₋₆CH=CH(CH₂)₁₋₆M,
- (CH₂)₀₋₆SO₂-N(R₃)-(CH₂)₀₋₆M,
- (CH₂)₀₋₆N(R₃)-(CH₂)₀₋₆N(R₃)-(CH₂)₀₋₆)-CO-M,
- N(R₃)-(CH₂)₁₋₇N(R₃) (CH₂)₁₋₆M,
- CH₂)₁₋₆-N(R₃)(CH₂)₁₋₇N(R₃)(CH₂)₀₋₆M, ou
- (CH₂)₀₋₆-N(R₃)-N(R₃)-(CH₂)₀₋₆M,
où un atome de carbone d'un groupe CH₂ peut être substitué par un groupe alkyle à chaîne droite ou ramifiée ayant de 1 à 4 atomes de carbone.

8. Composé selon la revendication 1, qui est l'un des composés suivants : N-[N-[(1S)-1-(Hydroxycarbonyl)-3-phénylpropyl]-L -alanyl]-4-(2,4-disulfamoyl-5-chloroanilino)proline ou N-[N-[(1S)-1-(hydroxycarbonyl)-3)phényl-propyl]-L-alanyl]-4-(2,4-disulfamoyl-5-chlorophénylimino)proline ou N-[N-[(1S)-1-(hydroxycarbonyl)-3-phénylpropyl]-L-alanyl]-4-(2-chloro-4-amino-5-sulfamoyl-phénylsulfonylimino)proline ou acide N-[N-[(1S)-1-(hydroxycarbonyl)-3-phénylpropyl]-L-alanyl]-3-(2,4-di-sulfamoyl-5-chloroanilino)-2,3-dihydroindole-2-carboxylique ou (4S)-1-((2S)-2-(N-(1S)-1-hydroxycarbonyl-3-phénylpropyl)amino)-propionyl)-4-(chloro-2-(furfuryl-amino)-5-sulfonamidobenzoyl)aminoproline ou 1-((2S)-2-(N-((1S)-1-hydroxycarbonyl-3-phényl-propyl)amino)-propionyl)-4-(2,3-dichloro-4-(carboxyméthoxy)benzoyl)-méthylène-proline ou 1-((2S)-2-(N-((1S)-1-hydroxycarbonyl-3-phényl-propyl)amino)-propionyl-4-éthoxy-4-(5-chloro-2,4-disulfonamidophényl)-aminoproline ou N-[N-[(1S)-1-hydroxycarbonyl-3-phénylpropyl]-L-alanyl]-4-[2-(6-chloro-7-sulfamoyl-3,4-dihydro-2H-1,2,4-benzo-thiadiazine-1,1-dioxyde-3-yl)éthoxy]proline ou (4R)-4-[5-(sulfamoyl)-4-chloro-2-[(2-furannylméthyl)amino]-benzoxy]-N-[N-[(1S)-1-(hydroxycarbonyl)-3-phénylpropyl]-L-alanyl]-L-proline ou ester éthylique de la (4R)-4-[5-(sulfamoyl)-4-chloro-2-[(2-furannylméthyl)amino]-benzoxy]-N-[N-(1S)-1-(éthoxy-carbonyl)-3-phénylpropyl]-L-alanyl]-L-proline ou N-[N-[(1S)-1-hydroxycarbonyl-3-phénylpropyl]-L-alanyl]-4-[((6-chloro-2-1,2,4-benzothiadiazine-1,1-dioxyde-7-yl)-sulfonyl)amino]-L-proline ou N-[N-[(1S)-1-hydroxy-carbonyl-3-phénylpropyl]-L-alanyl]-4-[2-(3-sulfamoyl-4-chlorobenzoyl)hydrazin-1-yl]-L-proline ou N-[N-[(1S)-1-hydroxycarbonyl-3-phénylpropyl]-L-alanyl]-4-((N-méthyl-N-(6-chloro-7-sulfamyl-3,4-dihydro-2H-1,2,4-benzo-thiadiazine-1,1-dioxyde-3-yl)méthyl)amino)proline ou acide N-[N-[(1S)-1-(hydroxycarbonyl)-3-phénylpropyl]-L-alanyl]-3-(2,4-disulfamoyl-5-chloroanilino)-2,3-dihydroindole-2-carboxylique, ou leurs sels d'addition avec un acide, de métaux alcalin et de métaux alcalino-terreux, acceptables d'un point de vue pharmaceutique.

9. Préparation pharmaceutique comprenant un composé selon l'une quelconque des revendications 1 à 8, et un excipient acceptable d'un point de vue pharmaceutique.

10. Utilisation de la préparation pharmaceutique de la revendication 9 pour traiter l'hypertension ou le glaucome.

11. Procédé pour préparer un composé selon l'une quelconque des revendications 1 à 8, qui consiste à coupler un composé de formule (II) 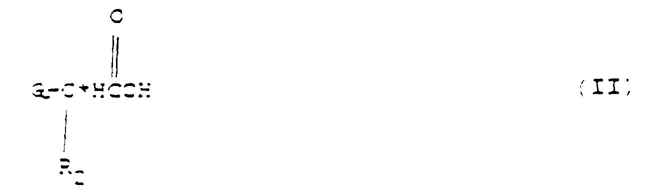 dans laquelle le groupe -COOH a été éventuellement converti en un groupe -COCl ou en le N-carboxyanhydride correspondant, avec un composé de formule (III) 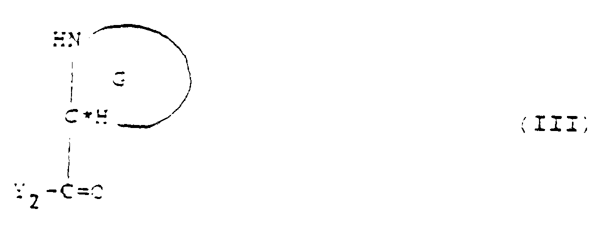 dans laquelle Q, R₂, Y₂ et G sont tels que définis dans la revendication 1, et éventuellement à former des sels du composé de formule (I).

## Revendications (Revendications pour l'(les) Etat(s) contractant(s) suivant(s): AT)

1. Procédé pour préparer un composé de formule (I) : 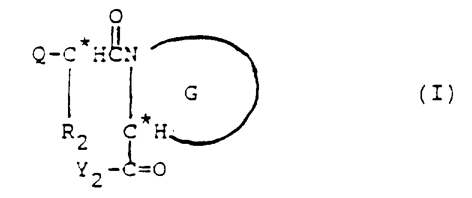 et ses sels, acceptables d'un point de vue pharmaceutique, d'addition avec un acide, de métaux alcalins et alcalino-terreux, où :
Q est Y₁-CO-C*H(R₁)NH-, R₁-CO-S(C*H(R₁))₀₋₁, ou HS-(C*HR₁)₀₋₁ ; où * désigne un carbone asymétrique ; Y₁ et Y₂, indépendamment l'un de l'autre, sont chacun -OH, -OR, ou -NR₁R₂ ;
G est 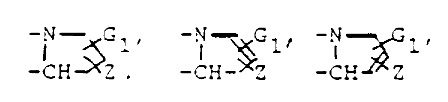 ou 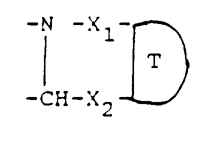
G₁ est H, -OH ou un radical alkyle en C₁₋₆ ou alcoxy en C₁₋₆ ;
X₁ et X₂, indépendamment l'un de l'autre, représentent chacun une liaison chimique ou un pont alkylène ayant 1, 2 ou 3 atomes de carbone, à condition que le noyau contenant X₁ et X₂ contienne de 4 à 6 atomes de carbone ; l'un des radicaux X₁ et X₂, ou les deux, sont éventuellement substitués par -OH, ou un radical alkyle en C₁₋₆ ou alcoxy en C₁₋₆ ; l'un des radicaux X₁ et X₂ est substitué par Z ; X₁ et X₂ sont autrement substitués par un hydrogène ; et T est un noyau hydrocarboné saturé, insaturé ou aromatique, ayant de 5 à 7 atomes de carbone ;
Z est =CH-(alk)-M, =CH-(alk)-CO-(alk)-M, =N-(alk)-M,
- (alk)-CH=CH-(alk)-M, =N-(alk)-SO₂M,
- (alk)-SO₂M, -(alk)-N(R₃)-(alk)-M, -(alk)-CO-(alk)-M,
- (alk)-N(R₃)-(alk)-CO-(alk)-M,
- (alk)-CO-(alk)-N(R₃)-(alk)-M, -(alk)-N(R₃)-SO₂-(alk)-M,
- (alk)-SO₂-N(R₃)-(alk)-M,
- (alk)-N(R₃)-(alk)-N(R₃)-(alk)-M,
- (alk)-N(R₃)-(alk)-N(R₃)-(alk)-CO-M, ou -O-(alk)-CO-M,
où (alk) est une liaison chimique, une chaîne alkylène de formule -(CᵢH₂ᵢ)-, ou encore une chaîne alkylène de formule -(C₁H₂ᵢ₋₁)-, qui est substituée par un groupe alkyle à chaîne droite ou ramifiée, ayant de 1 à 4 atomes de carbone, où i vaut de 0 à 6 ;
M est 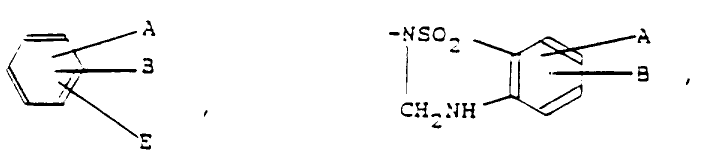 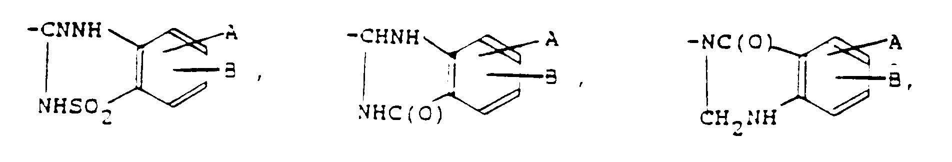 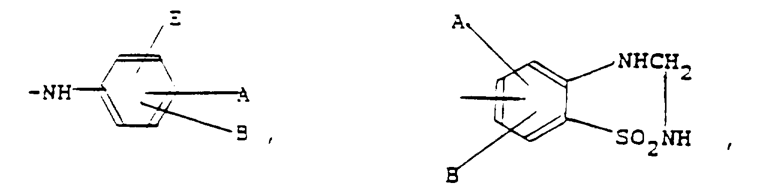 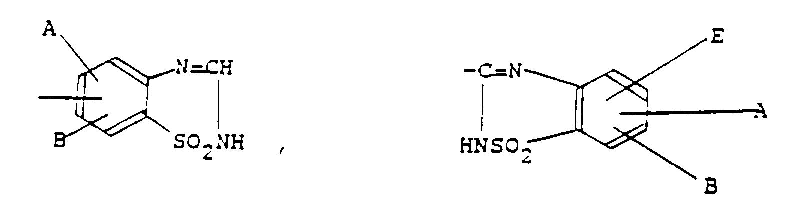 ou 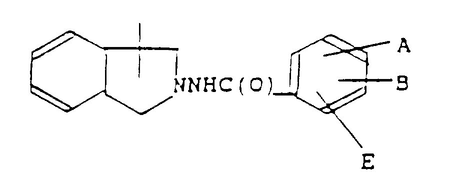
où A, B et E sont, indépendamment les uns des autres, H, un radical alkyle en C₁₋₆, phényle, benzyle, phénoxy, nitroalkylamino, alcanoylamino, alcanoylaminoalkyle, nitro, -OCH₂COOH, halogèno, hydroxy, -CF₃, -SR, -OR, -NR₁R₂, -CO-NR₁R₂, -CO-Y₁, -SO₂R, -SO₂-NR₁R₂ ou furfuryl-amino, du moment qu'au moins l'un des radicaux A et B n'est pas un hydrogène ;
R, R₁, R₂ et R₃, chacun indépendamment des autres, est un hydrogène ou un radical alkyle ayant de 1 à 8 atomes de carbone, aryle ayant jusqu'à 12 atomes de carbone, arylalkyle dans lequel le fragment aryle a jusqu'à 10 atomes de carbone et le fragment alkyle a de 1 à 6 atomes de carbone, cycloalkylaryle condensé ayant de 8 à 12 atomes de carbone, un groupe hétérocyclique, ou encore un groupe alkyle ayant de 1 à 6 atomes de carbone, qui est substitué par -NH₂, -NH-C(NH₂)=NH, ou 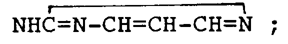 où R₁ et R₂ ne peuvent être des hydrogènes ;
où les groupes alkyle, cycloalkyle, aryle et arylcycloalkyle condensé peuvent porter des substituants choisis parmi l'ensemble comprenant les groupes alcoxy ayant de 1 à 6 atomes de carbone, -CF₃, -OH, -SH, halogèno, -NO₂ et -COOR ;
à condition que
quand le noyau G est saturé ou bicyclique, et Z est
- (alk)-N(R₃)-CO-phényl
- (alk)-N(R₃)-SO₂-phényl
- (alk)-N(R₃)-CH₂-phényl 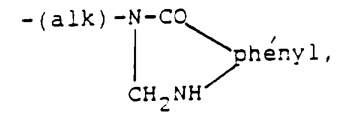 ou 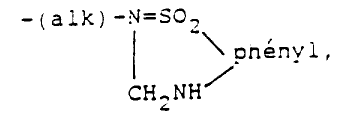
alors (alk) est un radical alkyle ramifié et/ou le fragment phényle est substitué par un substituant amino, furfurylamino, -OCH₂CO₂H ou deux groupes sulfamoyle. ;
qui consiste à coupler un composé de formule (II) 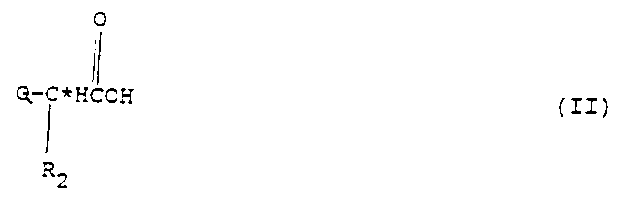
dans laquelle le groupe -COOH a été éventuellement converti en un groupe -COCl ou en le N-carboxyanhydride correspondant, avec un composé de formule (III) 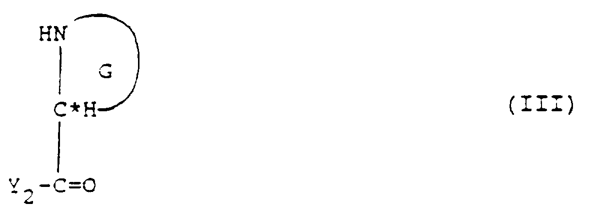
dans laquelle Q, R₂, Y₂ et G sont tels que définis ci-dessus, et éventuellement à former des sels du composé de formule (I)

2. Procédé selon la revendication 1, dans lequel
Z est =CH-(CH₂)ᵢCO-M, -(CH₂)ᵢ-CO-M, -(CH₂)ᵢ-N(R₃)-CO-M, -(CH₂)ᵢ-CO-N(R₃)-M, -(CH₂)ᵢ-SO₂M, -(CH₂)ᵢ-N(R₃)-M, -O-(CH₂)ᵢ-CO-M, -(CH₂)ᵢ-N(R₃)-SO₂-M-(CH₂)ᵢ-M,-N(R₃)-CH₂-(CH₂)ᵢ-N(R₃)-M, -O-(CH₂)ᵢ-M, =N-(CH₂)ᵢ-SO₂-M =N-(CH₂)ᵢ-M, =CH-(CH₂)ᵢ-M, ou -CH=CHM, où M, R₃ et i
sont tels que définis dans la revendication 1, à condition qu'un atome de carbone d'un maillon -(CH₂)ᵢ- peut être substitué par un groupe alkyle à chaîne droite ou ramifiée, ayant jusqu'à 3 atomes de carbone.

3. Procédé selon la revendication 1 ou 2, dans lequel Y₁ et Y₂, indépendamment l'un de l'autre, sont chacun -OH ou un radical alcoxy ayant de 1 à 8 atomes de carbone ou phényl-(alcoxy en C₁₋₆).

4. Procédé selon la revendication 3, dans lequel R₁ est H ou un radical alkyle ou phénylalkyle.

5. Procédé selon la revendication 4, dans lequel R₂ est H ou un radical alkyle ou aminoalkyle.

6. Procédé selon la revendication 5, dans lequel G est u n noyau pyrrole, tétrahydroquinoléine eu tétrahydroisoquinoléine.

7. Procédé selon la revendication 6, dans lequel
M est 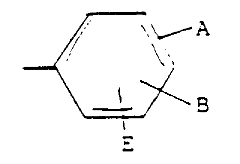
où A, B et E sont tels que définis dans la revendication 1.

8. Procédé selon l'une quelconque des revendications 1 à 7, dans lequel Z est
- (CH₂)₀₋₆CO-(CH₂)₁₋₆M,
- (CH₂)₀₋₆N(R₃)(CH₂)₁₋₆CO-(CH₂)₀₋₆M,
- (CH₂)₀₋₆N(R₃)-CO-(CH₂)₁₋₆M,
- (CH₂)₀₋₆CO-(CH₂)₁₋₆N(R₃)(CH₂)₀₋₆M,
- (CH₂)₀₋₆CO-N(R₃)(CH₂)₁₋₆M, -(CH₂)₀₋₆N(R₃)(CH₂)₁₋₆M,
- (CH₂)₀₋₆N(R₃)SO₂(CH₂)₁₋₆M, -(CH₂)₁₋₆O(CH₂)₀₋₆M,
- (CH₂)₀₋₆CH=CH(CH₂)₁₋₆M,
- (CH₂)₀₋₆SO₂-N(R₃)-(CH₂)₀₋₆M,
- (CH₂)₀₋₆N(R₃)-(CH₂)₀₋₆N(R₃)-(CH₂)₀₋₆)-CO-M,
- N(R₃)-(CH₂)₁₋₇N(R₃)(CH₂)₁₋₆M,
- CH₂)₁₋₆-N(R₃)(CH₂)₁₋₇N(R₃)(CH₂)₀₋₆M, ou
- (CH₂)₀₋₆-N(R₃)-N(R₃)-(CH₂)₀₋₆M,
où un atome de carbone d'un groupe CH₂ peut être substitué par un groupe alkyle à chaîne droite ou ramifiée ayant de 1 à 6 atomes de carbone.

9. Procédé selon la revendication 1, dans lequel le compose de la formule (I) est l'un des composés suivants : N-[N-[(1S)-1-(Hydroxycarbonyl)-3-phénylpropyl]-L-alanyl]-4-(2,4-disulfamoyl-5-chloroanilino)proline ou N-[N-[(1S)-1-(hydroxycarbonyl)-3)phénylpropyl]-L-alanyl]-4-(2,4-disulfamoyl-5-chloro-phénylimino)proline ou N-[N-[(1S)-1-(hydroxycarbonyl)-3-phénylpropyl]-L-alanyl]-4-(2-chloro-4-amino-5-sulfamoyl-phénylsulfonylimino)proline ou acide N-[N-[(1S)-1-(hydroxycarbonyl)-3-phénylpropyl]-L-alanyl]-3-(2,4-di-sulfamoyl-5-chloroanilino)-2,3-dihydroindole-2-carboxylique ou (4S)-1-((2S)-2-(N-(1S)-1-hydroxycarbonyl-3-phénylpropyl)amino)-propionyl)-4-(chloro-2-(furfuryl-amino)-5-sulfonamidobenzoyl)aminoproline ou 1-((2S)-2-(N-((1S)-1-hydroxycarbonyl-3-phényl-propyl)amino)-propionyl)-4-(2,3-dichloro-4-(carboxyméthoxy)benzoyl)-méthylène-proline ou 1-((2S)-2-(N-((1S)-1-hydroxycarbonyl-3-phényl-propyl)amino)-propionyl-4-éthoxy-4-(5-chloro-2,4-disulfonamidophényl)-aminoproline ou N-[N-[(1S)-1-hydroxycarbonyl-3-phénylpropyl]-L-alanyl]-4-[2-(6-chloro-7-sulfamoyl-3,4-dihydro-2H-1,2,4-benzo-thiadiazine-1,1-dioxyde-3-yl)éthoxy]proline ou (4R)-4-[5-(sulfamoyl)-4-chloro-2-[(2-furannylméthyl)amino]-benzoxy]-N-[N-[(1S)-1-(hydroxycarbonyl)-3-phénylpropyl]-L-alanyl]-L-proline ou ester éthylique de la (4R)-4-[5-(sulfamoyl)-4-chloro-2-[(2-furannylméthyl)amino]-benzoxy]-N-[N-(1S)-1-(éthoxy-carbonyl)-3-phénylpropyl]-L-alanyl]-L-proline ou N-[N-[(1S)-1-hydroxycarbonyl-3-phénylpropyl]-L-alanyl]-4-[((6-chloro-2-1,2,4-benzothiadiazine-1,1-dioxyde-7-yl)-sulfonyl)amino]-L-proline ou N-[N-[(1S)-1-hydroxycarbonyl-3-phénylpropyl]-L-alanyl]-4-[2-(3-sulfamoyl-4-chlorobenzoyl)hydrazin-1-yl]-L-proline ou N-[N-[(1S)-1-hydroxycarbonyl-3-phénylpropyl]-L-alanyl]-4-((N-méthyl-N-(6-chloro-7-sulfamyl-3,4-dihydro-2H-1,2,4-benzo-thiadiazine-1,1-dioxyde-3-yl)méthyl)amino)proline ou acide N-[N-[(1S)-1-(hydroxycarbonyl)-3-phénylpropyl]-L-alanyl]-3-(2,4-disulfamoyl-5-chloroanilino)-2,3-dihydroindole-2-carboxylique, ou leurs sels d'addition avec un acide, de métaux alcalin et de métaux alcalino-terreux, acceptables d'un point de vue pharmaceutique.

10. Procédé pour préparer une préparation pharmaceutique anti-hypertensive, qui consiste à formuler un composé ou un sel selon l'une quelconque des revendications 1 à 9, et un excipient acceptable d'un point de vue pharmaceutique.

11. Procédé pour préparer une préparation pharmaceutique anti-glaucome, qui consiste à formuler un composé ou un sel selon l'une quelconque des revendications 1 à 9, et un excipient acceptable d'un point de vue pharmaceutique.
